(19)

**Europäisches Patentamt**

**European Patent Office**

**Office européen des brevets**

(11) **EP 3 582 226 A1**

(12) **EUROPEAN PATENT APPLICATION**

(43) Date of publication:
18.12.2019 Bulletin 2019/51

(21) Application number: 18178028.9

(22) Date of filing: 15.06.2018

(51) Int Cl.:
*G16H 20/70* (2018.01)   *G16H 20/30* (2018.01)
*G16H 20/60* (2018.01)   *G16H 40/67* (2018.01)
*G16H 50/30* (2018.01)   *G16H 50/70* (2018.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **VAN KOLLENBURG, Johanna Wilhelmina Maria
5656 AE Eindhoven (NL)**

• **BOGERS, Sandra
5656 AE Eindhoven (NL)**
• **BUIL, Vincentius Paulus
5656 AE Eindhoven (NL)**
• **GEURTS, Lucas Jacobus Franciscus
5656 AE Eindhoven (NL)**
• **ELDERMAN, Joep
5656 AE Eindhoven (NL)**
• **VERBURG, Pepijn
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **AN APPARATUS AND METHOD FOR PROVIDING CONTEXT-BASED INTERVENTION**

(57)    There is provided a computer-implemented method for providing context-based healthcare intervention to a user. The method comprises acquiring (202) context information from a plurality of sources, updating (204), based on the acquired context information, a user profile associated with the user, wherein the user profile comprises a concern level value for each of a plurality of healthcare topics, selecting (206) one of the plurality of healthcare topics based on the concern level values in the user profile, and providing (208), based on the selected healthcare topic, an intervention recommendation to the user, wherein the intervention recommendation is associated with proposed information related to the selected healthcare topic or a proposed adjustment for a user device.

Figure 1

EP 3 582 226 A1

**Description**

FIELD OF THE INVENTION

The present disclosure relates to an apparatus and method for providing context-based healthcare intervention to a user. The present disclosure also relates to an apparatus and method for optimizing the provision of context-based healthcare intervention recommendations to a user.

BACKGROUND OF THE INVENTION

Changing user behavior in order to achieve health benefits has always been challenging, in particular when permanent health benefits are desired. User adherence to predefined static schedules that work towards a goal, be it exercise or diet, is traditionally low. Diet coaches and personal trainers are usually more effective, because they adjust their strategies based on the characteristics, the preferences, and the progress of the individual.

There are currently some known solutions for building digital coaching solutions that tailor intervention strategies based on a user, and they typically involve interacting with the user via a graphical user interface. These solutions, however, usually focus on achieving one personal health target (e.g. losing weight or running a marathon) only for a specific individual, rather than achieving multiple personal health targets for multiple users at the same time. There are also a number of aspects that currently known solutions do not adequately address.

SUMMARY OF THE INVENTION

As noted above, there are a number of disadvantages associated with the currently available solutions for providing intervention relating to healthcare coaching and guidance to a user or multiple users, for example for a number of family members in a home environment.

For example, presently known systems do not take into account of the fact that the relevance of coaching content to the user may vary quickly over time due to changes in the health context of the user. Also, it is often unclear at the beginning how to personally coach users in an effective and acceptable way, and therefore a few different strategies may need to be explored - this need for trialing different strategies is not considered in currently available solutions. Moreover, currently known systems also do not address the fact that the process of coaching requires a continuous dialogue between a user and the coaching system so as to gather contextual information that cannot be captured via sensor data alone.

It would therefore be advantageous to provide an improved method for providing context-based healthcare intervention to a user. The present disclosure relates to instantiation, monitoring and adaptation of personalized behavior change interventions based on personal dynamic interest profiles, in particular in the field of personal coaching systems, connected personal care devices and/or personal health devices, conversational user interfaces, and artificial intelligence.

To better address one or more of the concerns mentioned earlier, in a first aspect, a computer-implemented method for providing context-based healthcare intervention to a user. The method comprises: acquiring context information from a plurality of sources, wherein the context information is related to at least one of: an activity of the user, a physiological status, of the user, and an environment of the user; updating, based on the acquired context information, a user profile associated with the user, wherein the user profile comprises a concern level value for each of a plurality of healthcare topics, and the concern level value is indicative of a degree of interest and/or requirement of the user to receive support on the respective healthcare topic; selecting one of the plurality of healthcare topics based on the concern level values in the user profile; and providing, based on the selected healthcare topic, an intervention recommendation to the user, wherein the intervention recommendation is associated with proposed information related to the selected healthcare topic or a proposed adjustment for a user device.

In some embodiments, selecting a healthcare topic may comprise: selecting a healthcare topic with an associated concern level value higher than a first predetermined threshold; or determining, subsequent to updating the user profile, a change in concern level value for at least one of the plurality of healthcare topics, and selecting the healthcare topic if the respective determined change in concern level value is higher than a second predetermined threshold ; or selecting a healthcare topic that is relevant or similar to another healthcare topic with an associated concern level value higher than a predetermined threshold.

In some embodiments, the computer-implemented method may further comprise acquiring a persuasion style preference of the user for the selected healthcare topic. In these embodiments, providing an intervention recommendation may be further based on the acquired persuasion style preference of the user for the selected healthcare topic.

In some embodiments, the proposed adjustment for a user device may comprise at least one of: changing a setting of the user device, switching on the user device, and switching off the user device.

In some embodiments, the user profile may further comprise a performance level value for each of the plurality of healthcare topics. In these embodiments, providing an intervention recommendation may be further based on the performance level value for the selected healthcare topic in the user profile.

In some embodiments, the computer-implemented method may further comprise: receiving an input from the user indicating an acceptance or a rejection of the provided intervention recommendation. In these embodiments, if the received input indicates an acceptance, the method may further comprise executing the intervention recommendation, and if the received input indicates a rejection, the method may further comprise providing, based on the selected healthcare topic, a different intervention recommendation to the user.

In some embodiments, if the received input indicates an acceptance, the method may further comprise determining a change in effectiveness of the executed intervention over a predetermined time period. In these embodiments, the computer-implemented method may further comprise: determining a difference between the determined change in effectiveness of the executed intervention and an expected effectiveness index, wherein the expected effectiveness index represents an expected change in effectiveness of the executed intervention; and adjusting a parameter and/or a content of the executed intervention if the determined difference is larger than a predetermined threshold.

In some embodiments, if the received input indicates an acceptance, the method may further comprise determining an overall satisfaction score indicative of a degree of satisfaction of the user with the executed intervention, wherein determining an overall satisfaction score comprises: determining a progress satisfaction score indicative of a degree of satisfaction of the user with respect to a progress resulting from the executed intervention; determining a progress orientation score indicative of a degree of willingness of the user to accept a more radical versus gentle means of persuasion; determining a persuasion satisfaction score indicative of a degree of satisfaction of the user with a persuasion style associated with the executed intervention; and determining the overall satisfaction score based on the progress satisfaction score, the progress orientation score, and the persuasion satisfaction score.

In some embodiments, the method may further comprise: determining a difference between the overall satisfaction score and an expected satisfaction score, wherein the expected satisfaction score is indicative of a degree of expected satisfaction of the user with the executed intervention; and adjusting a parameter and/or a content of the executed intervention if the determined difference is larger than a predetermined threshold.

In some embodiments, the computer-implemented method may further comprise: acquiring a plurality of healthcare-related messages; determining a priority value for each of the plurality of healthcare-related messages based on at least one of the acquired context information and the selected healthcare topic; and providing at least one of the plurality of healthcare-related messages to the user based on the priority value.

In some embodiments, providing at least one of the plurality of healthcare-related messages may be further based on a burden indicator value, wherein the burden indicator value is indicative of a likelihood of overloading the user with excessive information.

In some embodiments, the computer-implemented method may further comprise, subsequent to providing a healthcare-related message to the user, updating the burden indicator value.

In a second aspect, there is provided a computer program product comprising a computer readable medium, the computer readable medium having computer readable code embodied therein, the computer readable code being configured such that, on execution by a suitable computer or processor, the computer or processor is caused to perform the method according to the first aspect.

In a third aspect, there is provided an apparatus for providing context-based healthcare intervention to a user. The apparatus comprises a processor configured to: acquire context information from a plurality of sources , wherein the context information is related to at least one of: an activity of the user, a physiological status, of the user, and an environment of the user; update, based on the acquired context information, a user profile associated with the user, wherein the user profile comprises a concern level value for each of a plurality of healthcare topics, and the concern level value is indicative of a degree of interest and/or requirement of the user to receive support on the respective healthcare topic; select one of the plurality of healthcare topics based on the concern level values in the user profile; and provide, based on the selected healthcare topic, an intervention recommendation to the user, wherein the intervention recommendation is associated with proposed information related to the selected healthcare topic or a proposed adjustment for a user device.

According to the aspects and embodiments described above, the limitations of existing techniques are addressed. In particular, the above-described aspects and embodiments enable healthcare intervention to be recommended and provided to a user based on context information that is acquired from a plurality of different sources. The embodiments described above offer balanced personal coaching on a plurality of health goals in an ecosystem of connected personal care devices and/or personal health devices and operating in a family household setting. In this way, the embodiments as described in the present disclosure allow:

- building of insight over time via data collection and a continuous natural language dialogue;
- careful dynamic interest profiling of each user, so that each user receives support for issues that are relevant to them;
- tailored dialogues based on context, user profiles, and persuasion style preferences; and

- personalized behavioral change strategies based on effectiveness and acceptance, combining device behavior with conversation-based coaching.

There is thus provided an improved method and apparatus for providing context-based healthcare intervention to a user. These and other aspects of the disclosure will be apparent from and elucidated with reference to the embodiment(s) described hereinafter.

BRIEF DESCRIPTION OF THE DRAWINGS

For a better understanding of the embodiments, and to show more clearly how they may be carried into effect, reference will now be made, by way of example only, to the accompanying drawings, in which:

Fig. 1 is a block diagram of an apparatus for providing context-based healthcare intervention to a user according to an embodiment;
Fig. 2 illustrates a method for providing context-based healthcare intervention to a user according to an embodiment;
Fig. 3 is a diagram illustrating a system for providing context-based healthcare intervention to a user according to an embodiment;
Fig. 4 is a diagram illustrating the Caring Home module of Fig. 3;
Fig. 5 is a schematic diagram of a hierarchical structure of a plurality of healthcare topics in a database;
Fig. 6 is a schematic diagram of a Care Module associated with a healthcare topic;
Fig. 7 is a schematic diagram of a user profile managing unit of the apparatus of Fig. 4;
Fig. 8 is a schematic diagram illustrating contextual user data in a user profile for a plurality of healthcare topics in a hierarchical structure;
Fig. 9 illustrates exemplary scales for each of a plurality of parameters in a user profile for a healthcare topic;
Fig. 10 is a flowchart illustrating a method associated with an activated Care Module, according to an embodiment;
Fig. 11 is a flowchart illustrated in a method associated with a deactivated Care Module, according to an embodiment;
Fig. 12 is a schematic diagram of a dialogue scheduling unit of the apparatus of Fig. 4;
Fig. 13 is a schematic diagram for the dialogue scheduling unit of Fig. 12; and
Fig. 14 is a finite state diagram representing the behavior of the dialogue handler of the dialogue scheduler of Fig. 12.

DETAILED DESCRIPTION OF EMBODIMENTS

As noted above, there is provided an improved apparatus and a method of operating the same which addresses the existing problems.

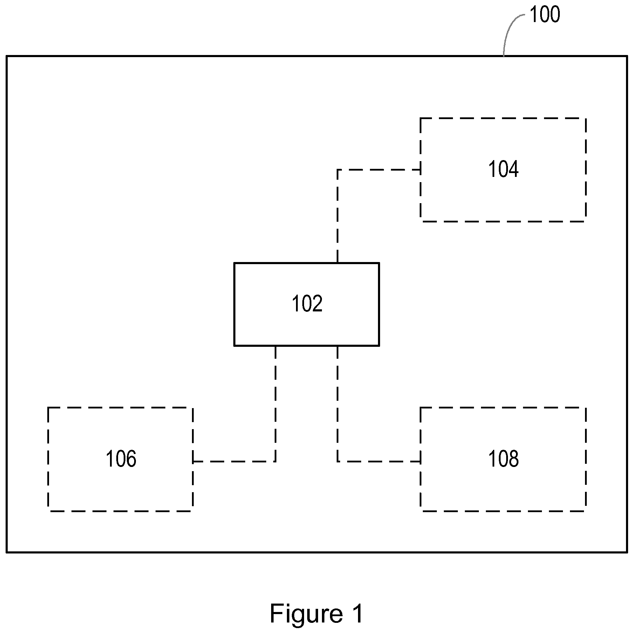

**Fig. 1** shows a block diagram of an apparatus 100 according to an embodiment, which can be used for providing context-based healthcare intervention to a user. Although the operation of the apparatus 100 is described below in the context of a single user, it will be appreciated that the apparatus 100 is capable of providing context-based healthcare intervention for a plurality of users.

As illustrated in Fig. 1, the apparatus comprises a processor 102 that controls the operation of the apparatus 100 and that can implement the method described herein. The processor 102 can comprise one or more processors, processing units, multi-core processor or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to acquire context information from a plurality of sources, wherein the context information is related to at least one of: an activity of the user, a physiological status, of the user, and an environment of the user. The plurality of sources may include a user device. For example, the context information may be usage information of a user device which is related to an activity performed by the user using the user device. A user device may be a personal care device (e.g. an electric tooth, a hair dryer, etc.) or a person health device (e.g. a weighing scale, an activity tracker, etc.).

Based on the acquired context information, the processor 102 is configured to update a user profile associated with the user, wherein the user profile comprises a concern level value for each of a plurality of healthcare topics, and the concern level value is indicative of a degree of interest and/or requirement of the user to receive support on the respective healthcare topic. The processor 102 is further configured to select one of the plurality of healthcare topics based on the concern level values in the user profile.

The processor 102 is also configured to provide, based on the selected healthcare topic, an intervention recommendation to the user, wherein the intervention is associated with proposed information related to the selected healthcare topic or a proposed adjustment for a user device.

In some embodiments, the apparatus 100 may further comprise at least one user interface 104. Alternative or in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. Alternatively or in addition, a user interface 104 may be configured to receive a user input. For example, a user interface 104 may allow a user of the apparatus 100 to manually enter instructions, data, or information. In these embodiments, the processor 102 may be configured to acquire the user input from one or more user interfaces 104.

The user interface 104 may be a user interface capable of simulating and/or providing natural language conversation with a user. Hence, in some embodiments, the user interface 104 may be referred to as a conversational user interface. The conversational user interface may be implemented as a chat bot on a mobile device (e.g. a smartphone) which is capable of providing a graphical user interface, or a voice-based virtual assistant in a smart speaker device. As mentioned above, in some embodiments the apparatus 100 may be capable of providing context-based intervention for a plurality of users. In some embodiments, the apparatus 100 may comprise a user interface 104 for each of the plurality of users. Alternatively, in some embodiments, the apparatus 100 may comprise a single user interface 104 for interacting with a plurality of users, e.g. multiple family members in a family.

A user interface 104 may be any user interface that enables the rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

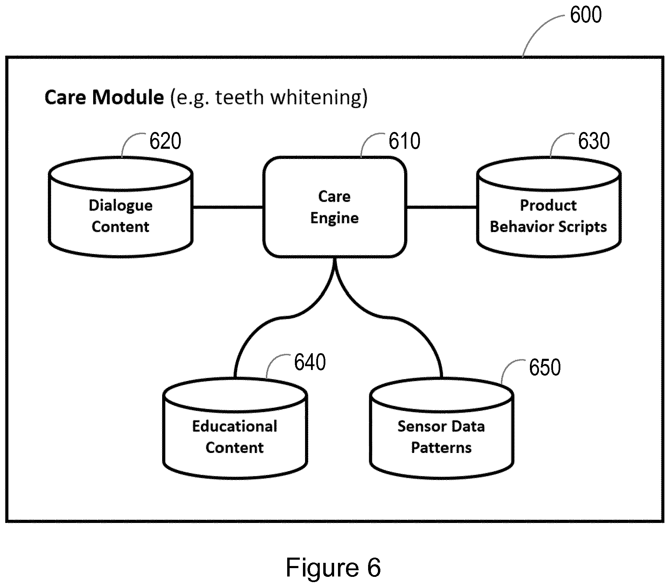

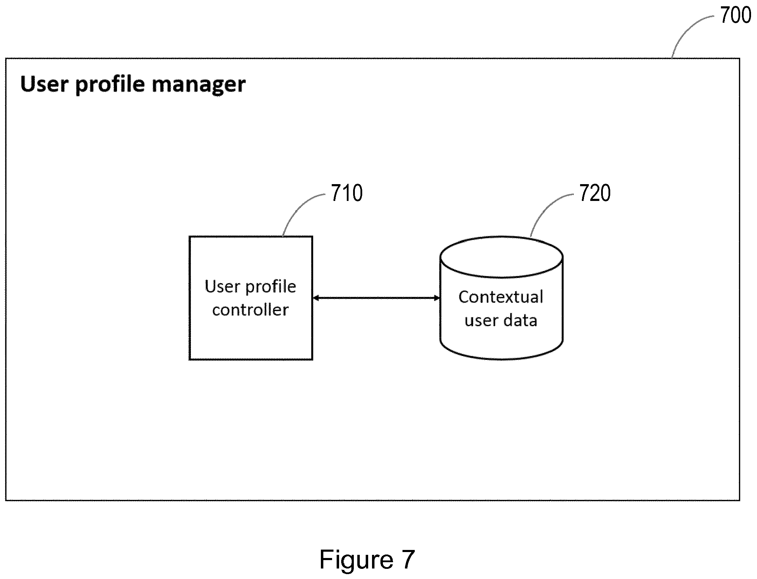

In some embodiments, the apparatus 100 may comprise a memory 106. Alternatively or in addition, one or more memories 106 may be external to (i.e. separate to or remote from) the apparatus 100. For example, one or more memories 106 may be part of another device. A memory 106 can be configured to store program code that can be executed by the processor 102 to perform the method described herein. A memory can be used to store information, data, signals and measurements acquired or made by the processor 102 of the apparatus 100. For example, a memory 106 may be used to store (for example, in a local file) a plurality of user profiles. The processor 102 may be configured to control a memory 106 to store the plurality of user profiles. Examples of memory in the context of the present disclosure may include a dialogue content storage unit (as shown in Fig. 6), a contextual user data storage unit (as shown in Fig. 7), etc.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with one or more user interfaces 104 wirelessly or via a wired connection. Similarly, the communications interface 108 may communicate with the one or more memories 106 wirelessly or via a wired connection.

It will be appreciated that Fig. 1 only shows the components required to illustrate an aspect of the apparatus 100 and, in a practical implementation, the apparatus 100 may comprise alternative or additional components to those shown.

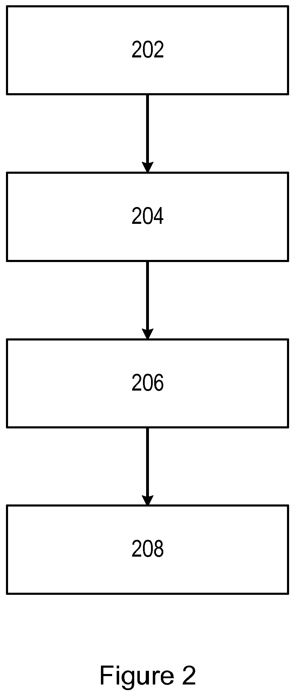

**Fig. 2** illustrates a computer-implemented method for providing context-based healthcare intervention to a user according to an embodiment. The illustrated method can generally be performed by or under the control of processor 102 of the apparatus 100.

With reference to Fig. 2, at block 202, context information is acquired from a plurality of sources. More specifically, context information may be acquired by the processor 102 of the apparatus 100. In some embodiments, context information may be acquired from one or more databases in a memory 106, which may be a memory of the apparatus 100 or a memory external to the apparatus 100, such as a memory in a user device (e.g. a personal care device or a personal health device, such as an electric toothbrush).

The acquired context information is related to at least one of: an activity of the user, a physiological status of the user, and an environment of the user. For example, the acquired context information may comprise one or more of: usage information of a user device (e.g. an electric toothbrush, such as a frequency of usage and/or a device setting during usage), location information (e.g. a current location of the user), weather information (e.g. of a current location of the user), time and/or date information (e.g. a current date and/or time), a physiological status of the user (e.g. whether the user is awake or asleep), and a subjective opinion based on a user input (e.g. the user indicating that they are interested in winding down). In some embodiments, the acquired context information may be related to one or more other users, in particular one or more other users of the apparatus 100 (e.g. other family members living in the same home environment). For example, the acquired context information may be related an activity of the other user.

As indicated above, the context information is acquired from a plurality of sources. For example, in an exemplary embodiment, the plurality of sources may include a user device, which may be a personal care device (e.g. devices related to dental care, hair care, skin care, etc.) or a personal health device (e.g. a weighing scale, an activity tracker, a sleep monitor, etc.), and also one or more sensors or actuators that are present in a home environment of the user or worn by the user.

Returning back to Fig. 2, at block 204, a user profile associated with the user is updated based on the context information acquired at block 202. The user profile may be acquired or retrieved by the processor 102 of the apparatus 100 from one or more databases in a memory 106, which may be a memory of the apparatus 100 or a memory external to the apparatus 100.

The user profile comprises a concern level value for each of a plurality of healthcare topics, and the concern level value is indicative of a degree of interest and/or requirement of the user to receive support on the respective healthcare topic.

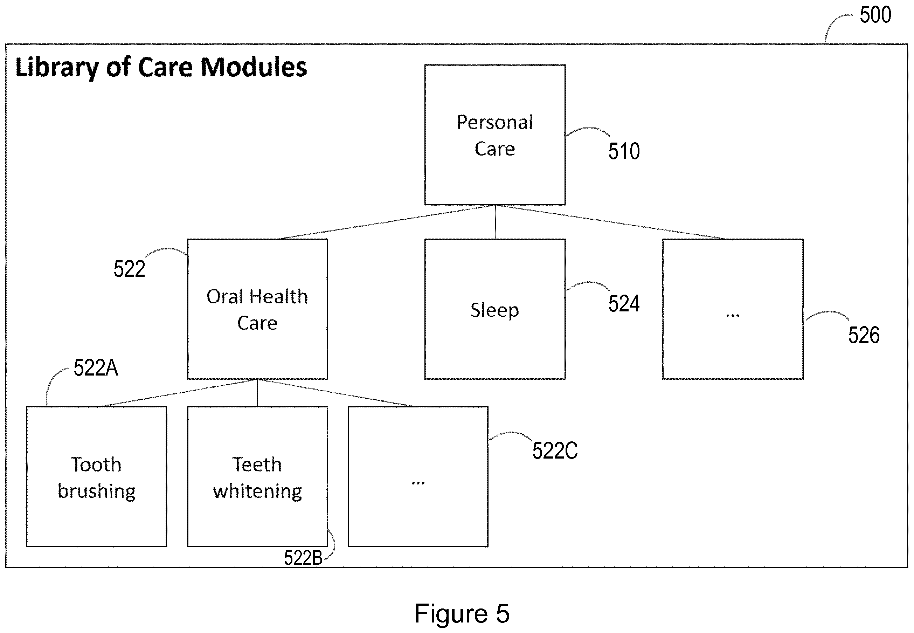

Each of the plurality of healthcare topics may be associated with a specific aspect of healthcare of the user, for example, tooth brushing. Moreover, in some embodiments, each of the plurality of healthcare topics may be organized in a hierarchical structure in a database. For example, the database may comprise, at a first level (i.e. a top level), "Personal Care" as a general healthcare topic. Under "Personal Care", at a second level, one or more specific healthcare topics may be provided, such as "Oral Health Care" and "Sleep". Furthermore, under each of the healthcare topics at the second level, one or more healthcare sub-topics may be provided at a third level. For example, under "Oral Health Care", healthcare sub-topics such as "Tooth brushing" and "Teeth whitening" may be provided. This hierarchical structure will be explained in more detail with reference to Fig. 5. Any of the healthcare topics or healthcare sub-topics may be generally referred to as a healthcare topic in the description herein. Moreover, as will explained in more detail in the following description, the selection of a healthcare topic in some embodiments may correspond to an activation of a Care Module associated with the particular selected healthcare topic.

As will be explained in more detail below, in some embodiments a concern level value may be represented as one of: "I'm fine", "It's on my mind", "I'm worried", "I need help", etc. which are respectively different levels of concern or interest in terms of severity and which dictate at least one of: a type of support to be provided to the user, how much support should be provided to the user, a content of the support to be provided to the user. This support is to be provided in the form of an intervention.

As described above with reference to block 202, the context information acquired at block 202 is related to at least one of: an activity of the user, a physiological status of the user, and an environment of the user. In some embodiments, the acquired context information may be associated with at least one of the plurality of healthcare topics, and in these embodiments, at block 204, the user profile may be updated with respect to the healthcare topic to which the acquired context information is associated.

For example, the acquired context information at block 202 may be related to a tooth brushing session performed by the user. The acquired context information in this case, therefore, may be associated with the healthcare topic "Tooth brushing". Subsequently, in this example, the user profile associated with the user may be updated with respect to the healthcare topic "Tooth brushing". Specifically, the concern level value for the healthcare topic "Tooth brushing" may be updated based on this acquired context information.

Additionally or alternatively, in some embodiments, the user profile may be updated with respect to a healthcare topic which has an implied relationship with the healthcare topic to which the acquired context information is associated. For example, the acquired context information at block 202 may be related to a tooth brushing session performed by the user. The acquired context information in this case, therefore, may be associated with the healthcare topic "Tooth brushing". Subsequently, in this example, the user profile associated with the user may be updated with respect to the healthcare topic "Sleep" due to an implied relationship between the healthcare topics "Tooth brushing" and "Sleep" (i.e. that users typically brush their teeth before going to sleep). Specifically, the concern level value for the healthcare topic "Sleep" may be updated based on this acquired context information.

In some embodiments, the user profile may be updated based on a user input. Specifically, in some embodiments at least one of the concern level values in the user profile may be updated based on a user input. For example, a concern level value for a particular healthcare topic may be changed from "I'm fine" to "It's on my mind" by the user via the user interface 104 of the apparatus 100, e.g. by unselecting "I'm fine" and selecting "It's on my mind" via a touchscreen input.

Returning back to Fig. 2, at block 206, one of the plurality of healthcare topics is selected based on the concern level values in the user profile. Specifically, one of the plurality of healthcare topics may be selected by the processor 102 of the apparatus 100.

In some embodiments, the selection of one of the plurality of healthcare topics may comprise selecting a healthcare topic with an associated concern level value higher than a first predetermined threshold.

Alternatively, in some embodiments, the selection of one of the plurality of healthcare topics may comprise determining, subsequent to updating the user profile, a change in concern level value for at least one of the plurality of healthcare topics, and selecting the healthcare topic if the respective determined change in concern level value is higher than a second predetermined threshold.

Alternatively, in some embodiments, the selection of one of the plurality of healthcare topics may comprise selecting a healthcare topic that is relevant or similar to another topic with an associated concern level value higher than a predetermined threshold.

Returning back to Fig. 2, at block 208, an intervention recommendation is provided to the user based on the healthcare topic selected at block 206. The intervention recommendation is associated with proposed information related to the selected healthcare topic or a proposed adjustment for a user device.

The proposed information related to the selected healthcare topic may comprise at least one of education and/or guidance information related to the selected healthcare topic and a reminder associated with the selected healthcare topic. The proposed adjustment for a user device may comprise at least one of: changing a setting of the user device, switching on the user device, and switching off the user device. In some embodiments, the intervention recommendation may be associated with multiple simultaneous or sequential interventions to be executed.

Moreover, in some embodiments, the proposed adjustment for a user device may be based on one or more selected product behavior scripts. In these embodiments, the computer-implemented method may further comprise selecting one or more product behavior scripts based on the acquired context information. For example, if it is determined from the acquired context information that the user is currently asleep or that another user within the vicinity is asleep, the processor 102 may be configured to select a product behavior script comprising instructions to adjust a setting of an electric toothbrush to disable acoustic feedback, so as to avoid disturbing the sleeping user.

In some embodiments, the intervention recommendation may be based on the concern level value of the selected healthcare topic. For example, if the selected healthcare topic is "sleep" and the associated concern level value is "I'm fine", the intervention recommendation may be associated with the provision of generic educational information, tips and tricks for falling asleep; if the associated concern level value is "I'm worried", the intervention recommendation may be associated with guidance information to help the user identify causes of difficulty falling asleep and providing a coaching program; if the associated concern level value is "I need help", the intervention recommendation may be associated with providing the user with real-time coaching, breathing exercise, and performing data gathering.

In some embodiments, the user profile may further comprise a performance level value for each of the plurality of healthcare topics, the performance level value being indicative of a quality of a user performance of an activity associated with a respective healthcare topic. In these embodiments, the provision of an intervention recommendation may be further based on the performance level value for the selected healthcare topic in the user profile. Moreover, the performance level value of the user for the selected healthcare topic may be updated based on a result achieved by the user in respect of an executed intervention (e.g. whether the user brushes their teeth) and/or a behavior of the user in respect of an executed intervention (e.g. how often the user brushes their teeth). For example, subsequent to providing an intervention recommendation at block 208 and receiving a user input indicating an acceptance of the intervention recommendation, the user profile may be updated based on a result score that is indicative of a degree of result achieved by the user in respect of the executed intervention.

In some embodiments, the computer-implemented method may further comprise acquiring a persuasion style preference of the user for the healthcare topic that is selected at block 206. The persuasion style preference provides guidance on a content of an intervention recommendation for the user and/or how an intervention should be delivered to the user. In these embodiments, the provision of an intervention recommendation at block 208 may be further based on the acquired persuasion style preference (e.g. "strong intervention, "careful intervention", "active intervention", or other descriptive preferences such as "kind", "friendly", "social", "drill sergeant" etc.) of the user for the selected healthcare topic.

For example, if the acquired persuasion style preference is "careful intervention", an audio coaching advice to be provided to the user (as part of the intervention to be executed) may be presented in a friendly tone of voice; if the acquired persuasion style preference is "strong intervention", the audio coaching advice to be provided to the user be presented in a "drill sergeant" style tone of voice. The tone of voice (or other manners of presentation of guidance or coaching advice) may additionally or alternatively be selected based on the concern level value for the selected healthcare topic.

Moreover, for each individual user, a persuasion style preference may be different for each of the plurality of healthcare topics. For example, for an individual user, the persuasion style preference for the healthcare topic "diet" may be "kind" whereas the persuasion style preference for the healthcare topic "sports" may be "drill sergeant". In some embodiments, a persuasion style preference for a healthcare topic may vary based on the acquired context information (e.g. time or date information). For example, for an individual user, the persuasion style preference for the healthcare topic "diet" may be "kind" if it is determined (e.g. by the processor 102) from the acquired context information that it is currently Saturday morning, and the persuasion style preference for the same healthcare topic (i.e. "diet") may be "social" if it is determined from the acquired context information that it is currently Saturday afternoon.

In some embodiments, acquiring a persuasion style preference of the user for the selected healthcare topic may comprise acquiring a persuasion style preference of the user for the selected healthcare topic from the acquired user profile. In this case, the user profile already comprises the persuasion style preference for the selected healthcare topic.

Alternatively, acquiring a persuasion style preference of the user for the selected healthcare topic may comprise deriving a persuasion style preference of the user for the selected healthcare topic based on a persuasion style preference of the user for a healthcare topic that is similar or relevant to the selected healthcare topic.

Alternatively, acquiring a persuasion style preference of the user for the selected healthcare topic may comprise selecting a random persuasion style preference from a plurality of persuasion style preferences in a database.

As mentioned above, in some embodiments the proposed adjustment for a user device (of the provided intervention recommendation) may be based on one or more selected product behavior scripts. In some of the embodiments where the computer-implemented method further comprises acquiring a persuasion style preference of the user for the selected healthcare topic, the selection of the one or more product behavior scripts may be based on the acquired persuasion style preference. For example, if the selected healthcare topic is "teeth whitening", the processor 102 may be configured to select a product behavior script comprising instructions to adjust a setting of an electric toothbrush as "strong whitening mode" if the acquired persuasion style preference is "drill sergeant", and the processor 102 may be configured to select a product behavior script comprising instructions to adjust a setting of an electric toothbrush as "gentle whitening mode" if the acquired persuasions style preference is "kind".

In some embodiments, the computer-implemented method may further comprise receiving an input from the user indicating an acceptance or a rejection of the provided intervention recommendation. This input may be received via the user interface 104 of the apparatus 100. For example, the user may indicate acceptance or rejection by clicking a virtual button displayed at the user interface 104.

In these embodiments, if the received input indicates an acceptance, the computer-implemented method may further comprise executing the intervention recommendation; and if the received input indicates a rejection, the computer-implemented method may further comprise providing a different intervention recommendation to the user based on the selected healthcare topic. In some embodiments, providing a different intervention recommendation to the user may be further based on an acquired persuasion style preference of the user for the selected healthcare topic.

Furthermore, if the received input indicates an acceptance, the method may further comprise determining a change in effectiveness of the executed intervention over a predetermined time period. In these embodiments, determining a change in effectiveness of the executed intervention over a predetermined time period may comprise: determining a first effectiveness score at the start of the predetermined time period; determining a second effectiveness score at the end of the predetermined time period; and determining a change in effectiveness of the executed intervention over a predetermined time period by calculating a difference of the first and second effectiveness scores over the predetermined time period.

In these embodiments, determining an effectiveness score (e.g. the first effectiveness score or the second effectiveness score) may be comprise: determining a result score indicative of a degree of result achieved by the user in respect of the executed intervention; determining an adherence score indicative of a degree of adherence of the user to the executed intervention; and determining the effective score by dividing the result score by the adherence score.

The effectiveness score quantifies the objective progress made by the user relative to an amount of effort that the user has put into adhering to the executed intervention. To explain in further detail, an effectiveness score may be determined according to the below equation:

$$E = \frac{R}{A} \hspace{4cm} (1)$$

where $E$ represents the effectiveness score, $R$ represents the results score, and $A$ represents the adherence score.

In some embodiments, the results score $R$ may be a value between 0 and 1 so as to represent, proportionally, what has been achieved by the user relative to a maximum achievable goal associated with the provided intervention recommendation and/or executed intervention. For example, if the intervention recommendation provided at block 208 is associated with a maximum achievable goal for the user to lose 10kg of weight in 6 months, and the user has only lost 8kg within the 6 months, then the processor 102 of the apparatus 100 may assign a value of 0.8 as the result score $R$.

In some embodiments, the adherence score $A$ may be a value between 0 and 1 so as to represent, proportionally, a degree of adherence of the user to the executed intervention. For example, if the proposed information associated with the intervention recommendation provided at block 208 (and therefore the executed intervention) includes advice to the user on the selected healthcare topic, and it is determined (e.g. by the processor or based on a user input) that the user has only put 50% of the advice into action, then the processor 102 of the apparatus may assign a value of 0.5 as the adherence score $A$.

The effectiveness score E is a metric of results relative to adherence. With a lower degree of adherence, a lower degree of result is expected. Also, if the user is ineffective, the results may fall short of the adherence of the user.

In some cases, the user can be very effective if the results are high even with a low degree of adherence.

Based on the above description, in some embodiments the change in effectiveness of the executed intervention over a predetermined time period may be determined according to the below equation:

$$G_e(t_n) = \frac{E(t_n)}{T_S} - \frac{E(t_{n-1})}{T_S} \qquad (2)$$

where $G_e(t_n)$ represents the change in effectiveness over the predetermined time period, $E(t_{n-1})$ represents the first effectiveness score, $E(t_n)$ represents the second effectiveness score, Ts represents the predetermined time period. The first effectiveness score $E(t_{n-1})$ and the second effectiveness score $E(t_n)$ may be determined using equation (1) as provided above. In some embodiments, the change in effectiveness over a predetermined time period may be referred to as "Growth in Effectiveness".

Although it is described above that in some embodiments a change in effectiveness over a predetermined time period (i.e. "Growth in Effectiveness") may be determined based on a first effectiveness score and a second effectiveness score, which are both in turn determined based on result scores and adherence scores, in alternative embodiments a change in effectiveness over a predetermined time period may be determined based, at least partly, on user input. In these alternative embodiments, the computer-implemented method may further comprise receiving user input indicating a perceived degree of change in effectiveness of the executed intervention over a predetermined time period, and determining a change in effectiveness over the predetermined time period based on the received user input.

In some embodiments, the computer-implemented method may further comprise acquiring sensor data associated with at least one of an activity performed by the user and a user device. In these embodiments, at least one of the result score and the adherence score may be determined based on the acquired sensor data. Specifically, at least one of the result score and the adherence score may be determined based on comparing the acquired sensor data with a predetermined sensor data pattern. Further details relating to sensor data pattern(s) are described below with reference to Fig. 6.

In some embodiments, the determined adherence score may be used for updating the concern level value of the selected healthcare topic. For example, if the determined adherence score is low, the processor 102 may be configured to update the concern level value (e.g. changing the value from "I'm fine" to "I need help"). Similarly, in some embodiments, the determined results score may be used for updating the concern level value of the selected healthcare topic. For example, if the determined results score is high, the processor 102 may be configured to update the concern level value (e.g. changing the value from "I'm not OK" to "I'm OK").

In some embodiments, the computer-implemented method may further comprise determining a difference between the determined change in effectiveness of the executed intervention (within the predetermined time period) and an expected effectiveness index. The expected effectiveness index represents an expected change in effectiveness of the executed intervention. After determining this difference, the computer-implemented method may further comprise adjusting a parameter and/or a content of the executed intervention if the determined difference is larger than a predetermined threshold.

In some embodiments, the difference between the determined change in effectiveness of the executed intervention and the expected effectiveness index may be compared against a predetermined threshold according to the below equation:

$$|G_e(t_n) - C_e(t_n)| > M_e \qquad (3)$$

where $G_e$ represents the change in effectiveness, $C_e(t_n)$ represents the expected effectiveness index, and $M_e$ represents a margin of error. The margin of error represents a maximum allowed deviation from an expected change in effectiveness that does not require further action, such as to provide a different intervention recommendation to the user.

Hence, in these embodiments, as long as the change in effectiveness does not differ substantially (i.e. staying within the margin of error) from the expected effectiveness index, no action is required in terms of intervention recommendation and intervention execution. However, if the difference between the change in effectiveness and the expected effectiveness index is larger than the margin of error (i.e. a predetermined threshold), a parameter and/or a content of the executed intervention may be adjusted (e.g. further changing a setting of a user device, or adapting a content of the coaching/guidance information provided to the user), or a different intervention recommendation may be provided. The provision of a different intervention recommendation may be based on a persuasion style preference of the user for the selected healthcare topic.

In some embodiments, if the received input from the user indicates an acceptance of the intervention recommendation provided at block 208, the computer-implemented method may further comprise determining an overall satisfaction score indicative of a degree of satisfaction of the user with the executed intervention.

In these embodiments, determining an overall satisfaction score may comprise: determining a progress satisfaction score indicative of a degree of satisfaction of the user with respect to a progress resulting from the executed intervention; determining a progress orientation score indicative of a degree of willingness of the user to accept a more radical versus gentle means of persuasion; determining a persuasion satisfaction score indicative of a degree of satisfaction of the user with a persuasion style associated with the executed intervention; and determining the overall satisfaction score based on the progress satisfaction score, the progress orientation score, and the persuasion satisfaction score.

In more detail, in some embodiments the overall satisfaction score may be determined according to the below equation:

$$S_t = \frac{S_{prog}V_{prog} + S_{pers}(1 - V_{prog})}{2} \tag{4}$$

where $S_t$ represents the overall satisfaction score, $S_{prog}$ represents the progress satisfaction score, $V_{prog}$ represents the progress orientation score, and $S_{pers}$ represents the persuasion satisfaction score.

In some embodiments, the progress satisfaction score $S_{prog}$ may be a value between 0 and 1 so as to represent, proportionally, a degree of satisfaction of the user with respect to a progress resulting from the executed intervention. The value assigned as the progress satisfaction score may be based on an interaction between the user and the apparatus 100. For example, the processor 102 may be configured to present a query to the user, via the user interface 104, regarding their satisfaction with respect to the resulting progress, and the value assigned as the progress satisfaction score may be based on an input by the user in response to this query.

In some embodiments, the progress orientation score $V_{prog}$ may be a value between 0 and 1 so as to represent a degree of willingness of the user to accept a more radical versus gentle means of persuasion. Some users are more willing to accept more radical means of persuasion in order to achieve progress, and for these users the progress orientation score will be relatively higher. In some embodiments, the progress orientation score may be stored in a context user data storage unit.

In some embodiments, the persuasion satisfaction score $S_{pers}$ may be a value between 0 and 1 so as to represent a degree of satisfaction of the user with a persuasion style associated with the executed intervention. For example, if the persuasion style associated with the intervention recommendation provided at block 208 is deemed too aggressive by the user (which may be indicated in a user feedback through the user interface 104), this may be reflected by a low value of the persuasion satisfaction score. As another example, if the executed intervention is associated with a proposed adjustment for a user device and the user subsequently changes or corrects the setting of the user device from the proposed adjustment, this may be reflected by decreasing the value of the persuasion satisfaction score.

The computer-implemented method may further comprise determining a difference between the overall satisfaction score and an expected satisfaction score. The expected satisfaction score is indicative a degree of expected satisfaction of the user with the executed intervention, and may be determined based on the progress orientation score. Furthermore, subsequent to determining the difference between the overall satisfaction score and the expected satisfaction score, the computer-implemented method may further comprise adjusting a parameter and/or a content of the executed intervention if the determined difference is larger than a predetermined threshold.

In some embodiments, Intelligent Agent techniques may be used for learning and/or estimating at least one of a persuasion style preference, a parameter and/or a content associated with an intervention recommendation that contribute to increasing the degree of satisfaction of the user with the executed intervention (and therefore an increase in the overall satisfaction score). In these embodiments, the Intelligent Agent technique may comprise a model-based agent or a goal-based agent, wherein the model-based agent or goal-based agent comprises reward functionality so as to determine an effect of at least one of a persuasion style preference, a parameter and/or a content associated with an intervention recommendation on the overall satisfaction score. Furthermore, the provision of an intervention recommendation at block 208 may be based on this determined effect.

It will be appreciated that in alternative embodiments, the overall satisfaction score may be determined based on different factors. For example, the overall satisfaction score may be determined based on a user input indicating a general degree of satisfaction with respect to the provided intervention recommendation.

In some embodiments, if the received input from the user indicates a rejection of the intervention recommendation provided at block 208, the computer-implemented method may further comprise updating the user profile with respect to a persuasion style preference.

In some embodiments, the computer-implemented method may further comprise: acquiring a plurality of healthcare-related messages; determining a priority value for each of the plurality of healthcare-related messages based on at least one of the acquired context information and the selected healthcare topic; and providing at least one of the plurality of healthcare-related messages to the user based on the priority value.

In these embodiments, at least one of the plurality of healthcare-related messages may be acquired from a memory 106 of the apparatus. Also, in these embodiments, at least one of the plurality of healthcare-related messages may be related to the healthcare topic selected at block 206.

In some embodiments, providing at least one of the plurality of healthcare-related messages may be further based on a burden indicator value. The burden indicator value may be indicative of a likelihood of overloading the user with excessive information. In these embodiments, the computer-implemented method may further comprise, subsequent to providing a healthcare-related message (i.e. at least one of the plurality of healthcare-related messages) to the user, updating the burden indicator value. Specifically, the processor 102 of the apparatus 100 may be configured to update the burden indicator value.

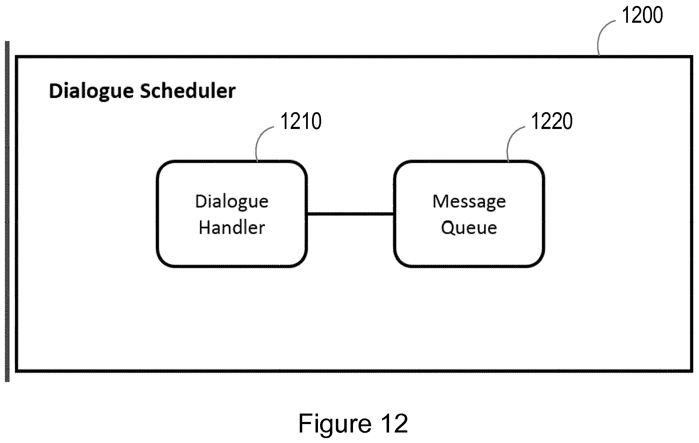

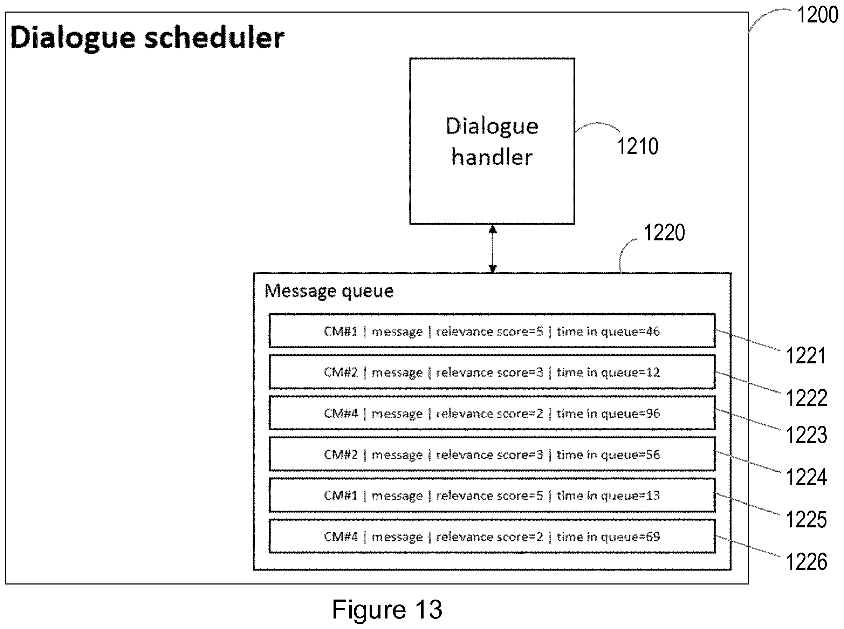

In some embodiments, the burden indicator value may be used by a dialogue handling unit in a system to determine whether a healthcare-related message is to be presented to the user. More detailed description relating to the dialogue handling unit is included below with reference to Fig. 12 and Fig. 13.

In some embodiments, updating the burden indicator value may comprise: increasing the burden indicator value every time a healthcare-related message is presented to the user (e.g. +1 unit for any message, or other values dependent on the healthcare topic to which the message is associated and/or a length of the message); and/or decreasing the burden indicator value in accordance with a predetermined rate or scale (e.g. -1 unit per minute).

In some embodiments, updating the burden indicator value may comprise updating the burden indicator value based on the acquired context information. For example, if it is determined from the acquired context information that the user is brushing their teeth (e.g. based on data received from an electric toothbrush of the user), the burden indicator value may be decreased to indicate that the user is less likely to be overloaded with excessive information if they were to be presented with multiple healthcare-related messages. Similarly, for example, if it is determined from the acquired context information that the user is applying makeup, the burden indicator value may be increased to indicate that the user is more likely to be overloaded with excessive information. Moreover, depending on a current date or a current time (which may also be determined from the acquired context information), the extent to which the burden indicator value is increased or decreased may differ, e.g. -5 unit if the tooth brushing session is in the morning, and -10 unit if the tooth brushing session is in the evening.

In some embodiments, a respective burden indicator value may be provided for each of the plurality of healthcare topics. In these embodiments, providing at least one of the plurality of healthcare-related messages may be based on a respective burden indicator value to which the at least one of the plurality of healthcare-related messages is associated.

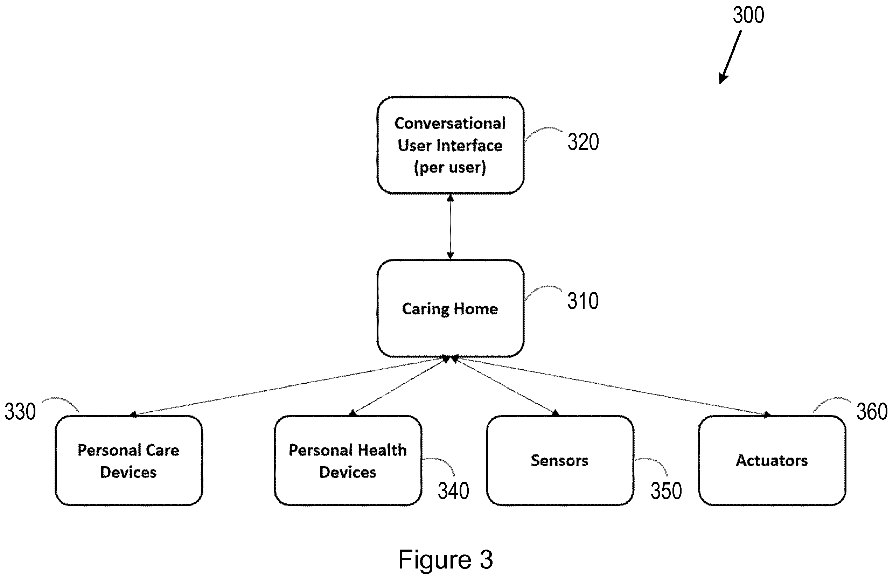

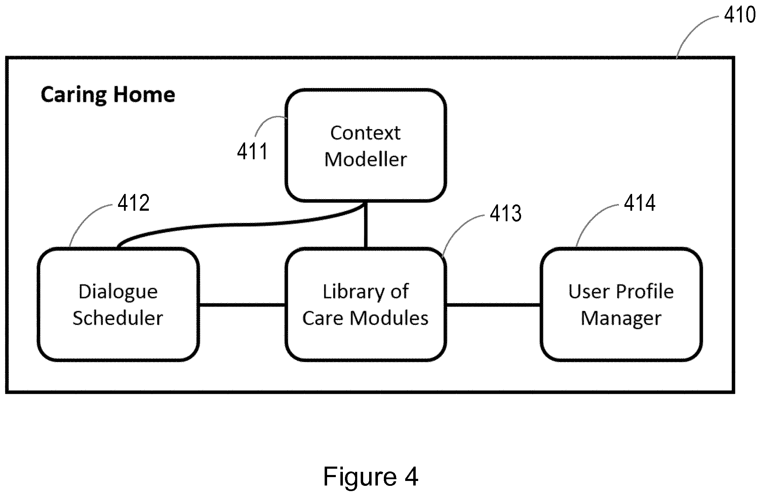

**Fig. 3** is a diagram illustrating a system 300 for providing context-based healthcare intervention to a user according to an embodiment, and **Fig. 4** is a diagram illustrating the Caring Home module of the system of Fig. 3.

As illustrated in Fig. 3, the system 300 comprises a Caring Home module 310, a conversational user interface 320, one or more personal care devices 330, one or more health devices 340, one or more sensors 350, and one or more actuators 360.

In the present embodiment, the Caring Home module 310 may be implemented in the system 300 inside a home environment and/or in a computing cloud. The Caring Home module 310 is configured to interact with one or more users via the conversational user interface (CUI) 320, which as mentioned with reference to Fig. 1 may be embodied in the form of a chat bot on a mobile device (through a graphical user interface, for example) or a voice-based virtual assistant in a smart speaker device. Moreover, in some embodiments, the Caring Home module 310 may be embodied in the form of an apparatus, such as the apparatus 100 as described with reference to Fig. 1.

The Caring Home module 310 is also further configured to interact with the one or more users via the one or more personal care devices 330 (e.g. devices related to dental care, hair care, skin care, etc.), and/or the one of more personal health devices 340 (e.g. a weighing scale, an activity tracker, a sleep monitor, etc.), and/or the one or more sensors 350, and/or the one or more actuators 360. In some embodiments, the one or more sensors 350 and the one or more actuators 360 may be present in the home environment of the one or more users, or being worn by the one or more users.

As shown in Fig. 4, the Caring Home module 410, which is equivalent to the Caring Home module 310 as illustrated in Fig. 3, comprises a context modelling unit 411, a dialogue scheduling unit 412, a healthcare topic database 413, and a user profile managing unit 414. In some cases, the context modelling unit 411 may be referred to as a "context modeler", the dialogue scheduling unit 412 may be referred to as a "dialogue scheduler", the healthcare topic database 412 may be referred to as a "library of Care Modules", and the user profile managing unit 414 may be referred to as a "user profile manager".

The context modelling unit 411 is configured to determine and/or monitor a context associated with each of the one or more users. This may be carried out in terms of a plurality of healthcare topics that are contained in the healthcare topic database 413. Examples of a context may include: brushing teeth, asleep, awake, etc. In some embodiments, the operation of the context modelling unit 411 may be implemented in a processor of an apparatus (e.g. the processor 102 of the apparatus 100 of Fig. 1).

The dialogue scheduling unit 412 is configured to receive conversational content (e.g. one or more healthcare-related messages) from the healthcare topic database 413, and to prioritize and balance the received conversational content before providing the conversational content to the one or more users. The dialogue scheduling unit 412 may be configured to perform such prioritization and balance based on a context determined by the context modelling unit 411 and the interests and/or requirements of the one or more users. The interest and/or requirements of a user may be indicated in a user profile associated with the user. More detailed explanation relating to the dialogue scheduling unit 412 is provided below with respect to Fig. 12 to 14.

The healthcare topic database 413 comprises a plurality of healthcare topics that are organized in a hierarchical structure. Examples of healthcare topics may include: tooth brushing, teeth whitening, improving sleep, oral health care, etc. In some embodiments, selection of one of the plurality of healthcare topics may correspond to an activation of a Care Module associated with the selected healthcare topic. When a Care Module associated with the selected healthcare topic is activated, coaching support on the respective healthcare topic may be delivered. This will be explained in more detail with reference to Fig. 5 below.

The user profile managing unit 414 is configured to manage and maintain one or more user profiles which are each respectively associated with the one or more users. The user profile managing unit 414 can be regarded as providing the functionality of maintaining a model of the interests and preferences of the one or more users with respect to the plurality of healthcare topics contained in the healthcare topic database 413. In some embodiments, the user profile managing unit 414 may further comprise a user profile control unit and a contextual user storage unit. This will be explained in more detail with reference to Fig. 7.

It will be appreciated that Fig. 3 and Fig. 4 only show the components required to illustrate an aspect of the system 300 and the Caring Home module 410, and in a practical implementation, the system 300 and/or the Caring Home module 410 may comprise alternative or additional components to those shown.

**Fig. 5** is a schematic diagram of a hierarchical structure of a plurality of healthcare topics in a database, such as the healthcare topic database 413 of Fig. 4. As indicated above, each of the healthcare topics may be selected, and the selection of a healthcare topic may correspond to an activation of a Care Module associated with the selected healthcare topic. Therefore, in some cases, for example in Fig. 5, the plurality of healthcare topics may be referred to as a "library of Care Modules". It will be appreciated that the plurality of healthcare topics shown in Fig. 5 are provided as examples, and in alternative embodiments, more healthcare topics or fewer healthcare topics may be represented.

In the present embodiment, each of the plurality of healthcare topics may be associated with a specific aspect of healthcare of the user. As shown in Fig. 5, the database comprises, at a first level (i.e. a top level), "Personal Care" 510 as a general healthcare topic. Under "Personal Care", at a second level, one or more further, more specific, healthcare topics may be provided, such as "Oral Health Care" 522 and "Sleep" 524, both of which falling under the more general topic of "Personal Care" 510. Additional healthcare topics may also be provided at the second level, which are represented by the box labelled 526.

Furthermore, under each of the healthcare topics 522, 524, 526 at the second level, one or more healthcare sub-topics may be provided at a third level. This is shown in Fig. 5, where healthcare sub-topics such as "Tooth brushing" 522A and "Teeth whitening" 522B may be provided under "Oral Health Care". Additional healthcare sub-topics may also be provided under "Oral Health Care" 522, which are represented by the box labelled 522C. Similarly, although not shown in the drawing, healthcare sub-topics such as "Effective Eating" and "Activity Routines" may be provided at the third level, under the healthcare topic "Sleep" 524.

Therefore, in embodiments where the plurality of healthcare topics organized in a hierarchical structure as shown in Fig. 5, the selection of a healthcare topic may be further optimized by allowing the selection of a healthcare topic that is relevant to a current context. For example, during a tooth brushing session of a user, it may be determined that the current context is associated with "Tooth Brushing" 522A. Based on this, the healthcare topic "Tooth brushing" 522A may be selected or a relevant healthcare topic (e.g. "Teeth whitening" 522B) may be selected. Additionally or alternatively, the healthcare topic "Oral Health Care" 522 may be selected since it can also be considered as a relevant healthcare topic, being in a directly higher level in the hierarchical structure. It will be appreciated that any of the healthcare topics or healthcare sub-topics may be generally referred to as a healthcare topic in the description herein.

**Fig. 6** is a schematic diagram of a Care Module 600 associated with a healthcare topic, which can be activated based on context by selecting the corresponding healthcare topic.

As shown in Fig. 6, the Care Module 600 comprises a Care Engine 610, a dialogue content storage unit 620, a product behavior script storage unit 630, an educational content storage unit 640, and a sensor data pattern storage unit 650.

The dialogue content storage unit 620 is configured to store dialogue content associated with a respective healthcare topic, which is used for the generation of conversations so as to allow collection of subjective opinion(s) and interest levels of a user on the respective healthcare topic and to provide coaching to a user on the respective healthcare topic.

The product behavior script storage unit 630 is configured to store product behavior scripts associated with a respective healthcare topic, which enable product behavior generation for intervention execution. In some embodiments, the product behavior scripts may include instructions to adjust a setting of a user device. For example, the product behavior scripts may dictate a certain setting for a user device, such as automatically setting a tooth brush to teeth whitening mode, and/or setting an alert sound effect if the user stops a tooth brushing session too early.

The educational content storage unit 640 is configured to store educational content associated with a respective healthcare topic, and the sensor data pattern storage unit 650 is configured to store one or more sensor data patterns associated with a respective topic. The sensor data patterns may enable meaningful data collection from sensors in relation to a Care Module associated with a healthcare topic. The sensor data patterns may be predefined based on domain knowledge. For example, domain knowledge for the healthcare topic "tooth brushing" may include a desired end result of "no cavities and no pain" and a corresponding desired user behavior "brush teeth at least twice a day for 2 minutes, covering all teeth". Domain knowledge may also be at least partly derived via Deep Learning (or other Artificial Intelligence) techniques using acquired sensor data.

In some embodiments, the sensor data patterns may comprise at least one of contextual data patterns and behavioral data patterns. By comparing acquired sensor data with the contextual data patterns, an activity performed by the user (e.g. whether the user has started or ended a tooth brushing session) or a physiological status of the user (e.g. just fell asleep, or just woke up) can be determined. The output of this determination may be transmitted to the context modelling unit. Also, by comparing acquired sensor data with the behavioral data patterns, a quality and/or a duration of an activity performed by the user can be determined (e.g. whether a session was short, medium, long, good, or bad).

The Care Engine 610 is configured to manage the behavior of the Care Module 600. Specifically, the Care Engine 610 is configured to activate and/or deactivate the Care Module 600, assess an interest level (which may be represented as a concern level value in some embodiments), and to determine the content and the product behavior scripts to be presented to a user. The operation of the Care Engine 610 may be based on a received user input via a user interface and/or received sensor data. The progress of a user is monitored and intervention recommendations may be adjusted based on performance and experience metrics defined in the Care Module 600. The operation of the Care Engine 610 may be structured with respect to a plurality of phases:

- User interest: a desired level of care (concern level value) may be determined (e.g. "I'm fine", "I'm worried", or "I need help"). The Care Engine 610 may be configured to determine if and what type of intervention to recommend to the user, and what outcome is likely to be expected by the user. The desired level of care (concern level value) may be stored inside the user profile managing unit.
- Information: educational content related to the healthcare topic may be provided to the user based on the desired level of care (concern level value).
- Proposal: a behavior change may be proposed in the form of an intervention recommendation. This may be based on a desired level of care (concern level value), a persuasion style preference, and/or a current physiological status of the user.
- Execution: the recommended intervention may be executed (e.g. product behavior, coaching dialogues)
- Progress evaluation: based on a set health target, and how the coaching is valued by the user, it is determined whether a different intervention strategy should be provided.
- End result evaluation: objective end result and the user's satisfaction with the end result is determined; end result may be evaluated based on acquired sensor data or by enquiring the user

It will be appreciated that Fig. 6 only shows the components required to illustrate an aspect of the Care Module 600 and, in a practical implementation, the Care Module 600 may comprise alternative or additional components to those shown. For example, in alternative embodiments the Care Module 600 may not comprise a dialogue content storage unit or an educational content storage unit.

**Fig. 7** is a schematic diagram of a user profile managing unit of the apparatus of Fig. 4. As described earlier with reference to Fig. 4, in some embodiments the user profile managing unit may further comprise a user profile control unit and a contextual user data storage unit. These components are shown in Fig. 7 as a user profile control unit 710 (which may be referred to as a "user profile controller") and a contextual user data storage unit 720 (labelled as "contextual user data" in Fig. 7).

In the present embodiment, the user profile control unit 710 is configured to manage a user interest model that can be updated over time for each of the one or more users, and the user interest model in this case may be represented by a plurality of concern level values for each of a plurality of healthcare topics in a database (e.g. the healthcare topic database 413 in Fig. 4). The contextual user data storage unit 720 is configured to store contextual user data.

In some embodiments, the contextual user data stored in the contextual user data storage unit 720 may be structured in the same way as the structure of the plurality of healthcare topics as shown in Fig. 5. This structure groups healthcare topics with similar objectives together. Also, this approach allows a user interest in a broader topic to trigger the selection of multiple healthcare topics (and therefore activation of multiple Care Modules) and allows the database/library to be more easily searchable and extendable. Under each of the plurality of healthcare topics, one or more of the following parameters may be stored:

- concern level value (which may be referred to as "interest level" or "level of care" in some embodiments)
- performance level value
- activation level value
- persuasion style preference
- burden indicator value

In some embodiments, the user profile control unit 710 may be configured to receive, for a user, an updated value for at least one of the concern level value, the performance level value, the activation level value, and the persuasion style preference, and to control the contextual user data storage unit 720 to store the received updated value. Moreover, the user profile control unit 710 may be configured to determine or estimate, for a respective healthcare topic, a value for at least one of the concern level, the performance level, activation level, and persuasion style preference, based on a value of a corresponding parameter of a relevant or similar healthcare topic. This determination or estimation may be based on an inference algorithm. In particular, in some embodiments, the user profile control unit 710 may be configured to determine or estimate a performance level and/or persuasion style preference for a respective healthcare topic based on a value of a corresponding parameter of at least one relevant healthcare topic that is lower down in the hierarchical structure.

For example, if it is determined that the concern level for the healthcare topic "tooth brushing" has a value of 3 (on a scale from 1 to 10) and the concern level for the health care topic "teeth whitening" has a value of 1 (on a scale from 1 to 10), then the user profile control unit 710 may be configured to determine or estimate the concern level value for the healthcare topic "oral health care" (which is one level higher than "tooth brushing" and "teeth whitening" in the hierarchical structure) as having a value of 2 (i.e. the average value of the concern level values of the two relevant healthcare topics). Other parameters, such as the persuasion style preference, can be estimated or determined in a similar way.

The concern level value of a healthcare topic may be used by a dialogue scheduling unit (e.g. the dialogue scheduling unit as will be described with reference to Fig. 12 and Fig. 13) to determine a priority value of a healthcare-related message to be presented to a user.

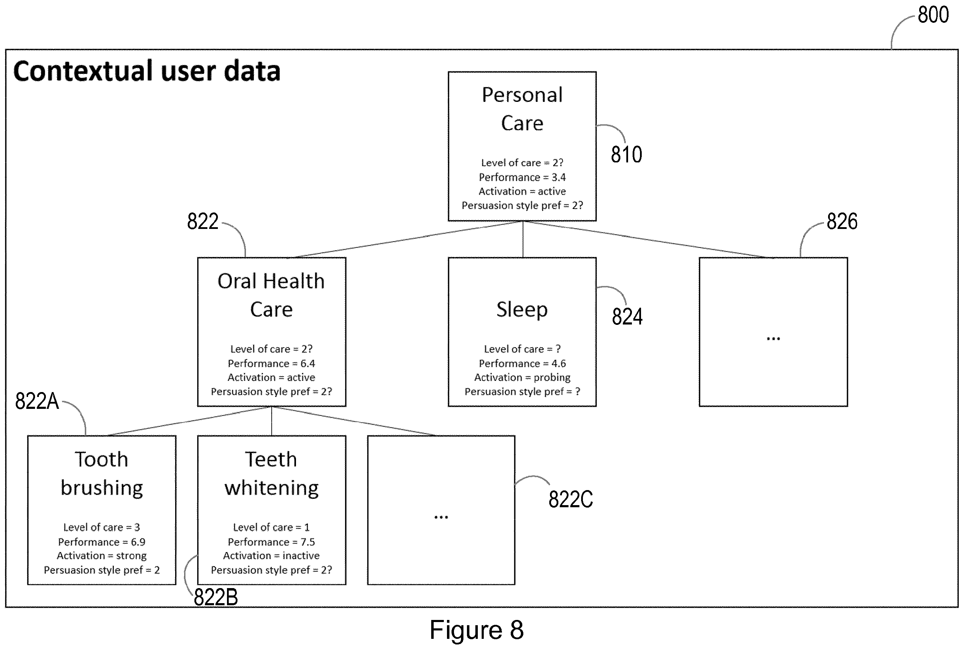

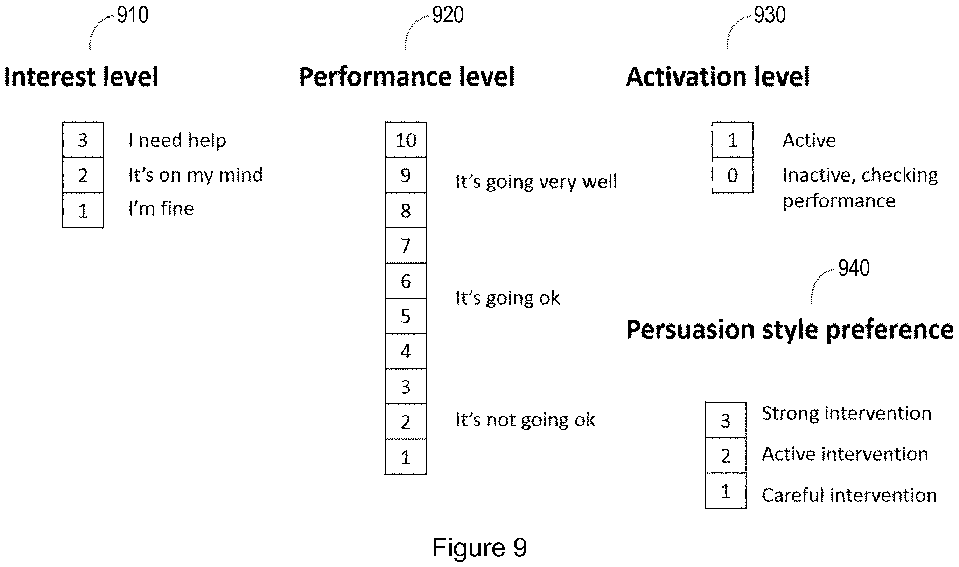

Fig. 8 is a schematic diagram illustrating contextual user data in a user profile for a plurality of healthcare topics in a hierarchical structure, and Fig. 9 illustrates exemplary scales for each of a plurality of parameters in a user profile for a healthcare topic.

The hierarchical structure of healthcare topics from the example in Fig. 5 is used in Fig. 8 to explain the information or data that can be associated with each of the plurality of healthcare topics. Similar to Fig. 5, the hierarchical structure in the present embodiment also comprises "Personal Care" 810, "Oral Health Care" 822, "Sleep" 824, "Tooth brushing" 822A, "Tooth whitening" 822B. Other healthcare topics under "Personal Care" are represented as 826 and other healthcare topics under "Oral Health Care" 822 are represented as 822C. In the present embodiment, the contextual user data (which can be stored in a contextual user data storage unit) comprises a concern level value (which may be referred to as "level of care"), a performance level value, an activation level value, and a persuasion style preference for each of the plurality of healthcare topics.

For example, referring to the healthcare topic "Tooth brushing" 822A, the concern level value is 3, the performance level value is 6.9, the activation level value is "strong", and the persuasion style preference is 2.

To explain the significance of each of the parameters (e.g. performance level value) associated with a healthcare topic, a plurality of exemplary scales is provided in Fig. 9. The plurality of exemplary scales includes a concern level value scale (which may be referred to as the "interest level" scale, as shown in the drawing) 910, a performance level value scale 920, an activation level value scale 930, and a persuasion style preference scale 940.

In this embodiment, the concern level value is provided in a scale of 1 to 3 in accordance to a degree of interest and/or requirement of the user to receive support on the respective healthcare topic, wherein a value of 1 corresponds to "I'm fine", a value of 2 corresponds to "It's on my mind", and a value of 3 corresponds to "I need help". Therefore, a concern level value of 3 for "Tooth brushing" 822A corresponds to "I need help".

In this embodiment, the performance level value is provided in a scale of 1 to 10 in accordance to a quality of a user performance of an activity associated with a respective healthcare topic. As shown in Fig. 9, a performance level value of 9 corresponds to "It's going very well", a performance level value between 5 and 6 corresponds to "It's going OK", and a performance level value of 2 corresponds to "It's not going OK". Therefore, a performance level value of 6.9 for "Tooth brushing" 822A can be interpreted as slightly less severe than "It's going OK".

In this embodiment, as indicated in the activation level value scale 930, the activation level value is either 1 or 0, wherein a value of 1 corresponds to "active" and a value of 0 corresponds to "inactive, checking performance". Although not shown in Fig. 9, in some embodiments the activation level can also take other values, for example the activation level value for "Tooth brushing 822A" is "strong", which is a level higher than "active" and indicates that stronger intervention should be provided to the user with respect to this particular healthcare topic. In some other embodiments, a possible activation level value may be "probing", which indicates that the Care Module associated with the respective healthcare topic in the process of determining whether it should be activated.

In this embodiment, the persuasion style preference is provided in a scale of 1 to 3 in accordance to a degree to strength of an intervention recommendation to be provided to the user, wherein a value of 1 corresponds to "careful intervention", a value of 2 corresponds to "active intervention", and a value of 3 correspond to "strong intervention".

It will be appreciated that in alternative embodiments, each of the concern level value scale 910, the performance level value scale 920, the activation level value scale 930, and the persuasion style preference scale 940 may be different, e.g. on a scale of 1 to 20 instead of a scale of 1 to 10. Furthermore, although it is described above that each of the concern level value scale, the performance level value scale, the activation level value scale, and the persuasion style preference scale comprises a plurality of discrete values, it will be appreciated that in alternative embodiments one or more of these scales may be a continuous scale instead of a discrete scale so as to allow more fine-grained monitoring and action.

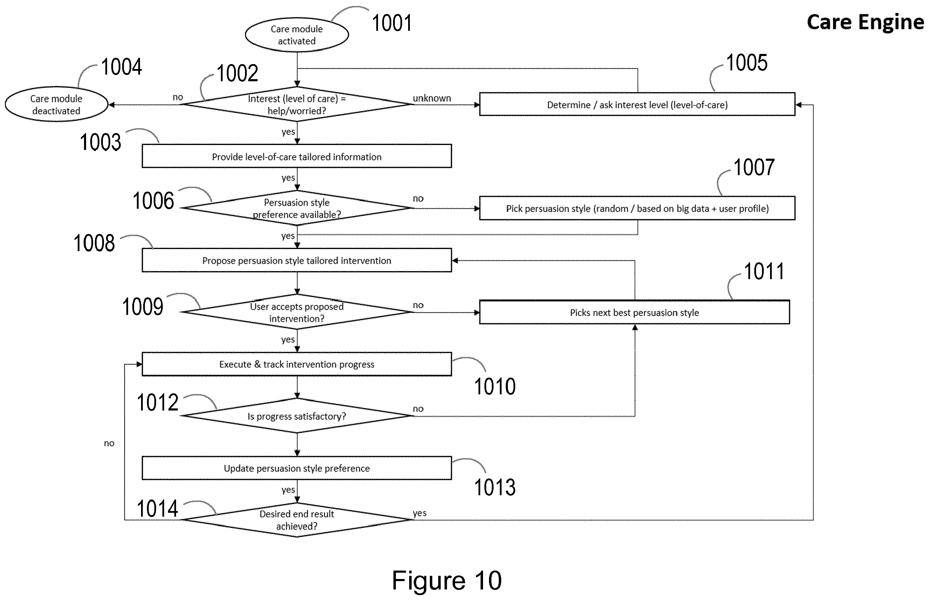

**Fig. 10** is a flowchart illustrating a method associated with an activated Care Module, according to an embodiment. In some embodiments, the method as shown in Fig. 10 may be performed by the processor 102 of the apparatus 100 as described with reference to Fig. 1. In some embodiments, the method may be performed by the Care Engine of an activated Care Module, such as the Care Engine 610 as illustrated in Fig. 6.

In the present embodiment, the method begins at step 1001, at which a Care Module is activated, which may be triggered by a selection of a particular healthcare topic. For example, if the healthcare topic "tooth brushing" is selected, the Care Module associated with the healthcare topic "tooth brushing" can be activated. Once the Care Module is activated, the method proceeds to step 1002, at which it is determined whether the concern level value of the selected healthcare topic (i.e. "interest (level of care)") is one of "I need help" or "I'm worried".

If it is determined at step 1002 that the concern level value is one of "I need help" or "I'm worried", the method proceeds to step 1003 where tailored information (e.g. educational content) is provided to the user based on the concern level value. In some embodiments, the tailored information may be provided via a conversational user interface. Moreover, in some embodiments, the tailored information may be retrieved from a dialogue content storage unit.

On the other hand, if it is determined at step 1002 that the concern level is not "I need help" or "I'm worried", then the method proceeds to step 1004 where the Care Module is deactivated. Alternatively, if it is determined at step 1002 that the concern level value is unknown, then the method proceeds to step 1005 where an concern level value for the healthcare topic associated with the activated Care Module is determined (e.g. by enquiring the user via a user interface), before returning back to step 1002 .

Subsequent to determining a concern level value at 1005, the method may return to step 1002, where it is determined whether the concern level value is "I need help" or "I'm worried".

Subsequent to providing tailored information at step 1003, the method proceeds to step 1006, where it is determined whether there is an available persuasion style preference associated with the healthcare topic corresponding to the activated Care Module.

If it is determined at step 1006 that there is an available persuasion style preference associated with the healthcare topic, the method proceeds directly to step 1009. Otherwise, if it is determined at step 1006 that there is no available persuasion style preference associated with the healthcare topic, the method proceeds to step 1007 where a persuasion style preference is either derived based on at least one of: big data, a user profile of the user, and a persuasion style preference of a similar healthcare topic, or selected in a random manner, before proceeding to step 1008.

At step 1008, an intervention recommendation is provided based on the available persuasion style preference. The intervention recommendation may comprise dialogue content (e.g. reminders, coaching and guidance) retrieved from a dialogue content storage unit, and/or product behavior script (e.g. a certain setting for a user device, such as automatically setting a tooth brush to teeth whitening mode, and/or setting an alert sound effect if the user stops a tooth brushing session too early) retrieved from a product behavior script storage unit. As indicated previously, the product behavior script may include instructions to adjust a setting of a user device.

If the intervention recommendation is accepted at step 1009, the method proceeds to step 1010. Otherwise, the method proceeds to step 1011 where a next best persuasion style is selected, and subsequently to step 1008 where a (different) intervention recommendation is provided based on the updated persuasion style preference.

At step 1010, an intervention is executed based on the intervention recommendation and a progress resulting from the executed intervention is tracked. In some embodiments, tracking of the progress may be based on determination of a change in effectiveness as described with reference to Fig. 2. If it is determined at step 1012 that the resulting progress is satisfactory, the method proceeds to step 1013, where the persuasion style preference for the healthcare topic associated with the activated Care Module is updated (e.g. by replacing with a currently selected persuasion style preference in the contextual user data stored in a contextual user data storage unit). Otherwise, the method returns to step 1011 where (another) next best persuasion style preference is selected and subsequently to step 1008 where another (different) intervention is provided based on the updated persuasion style preference.

After updating the persuasion style preference at step 1013, the method proceeds to step 1014 where it is determined whether a desired end result is achieved. In some embodiments, determining whether a desired end result is achieved may be based on determining of a result score as described with reference to Fig. 2. If it is determined at step 1014 that the desired end result is achieved, the method proceeds to step 1005 where (a new) concern level value for the healthcare topic associated with the activated Care Module is determined. If it is determined at step 1014 that the desired end result is not achieved, the method proceeds to step 1011 where (further) intervention is executed and a progress resulting from the (further) intervention is tracked.

It will be appreciated that in alternative embodiments the method may not comprise all of the steps described, and one or more steps as illustrated in Fig. 10 may be omitted. It will be also be appreciated that in alternative embodiments, the steps as illustrated in Fig. 10 may be performed in a different order.

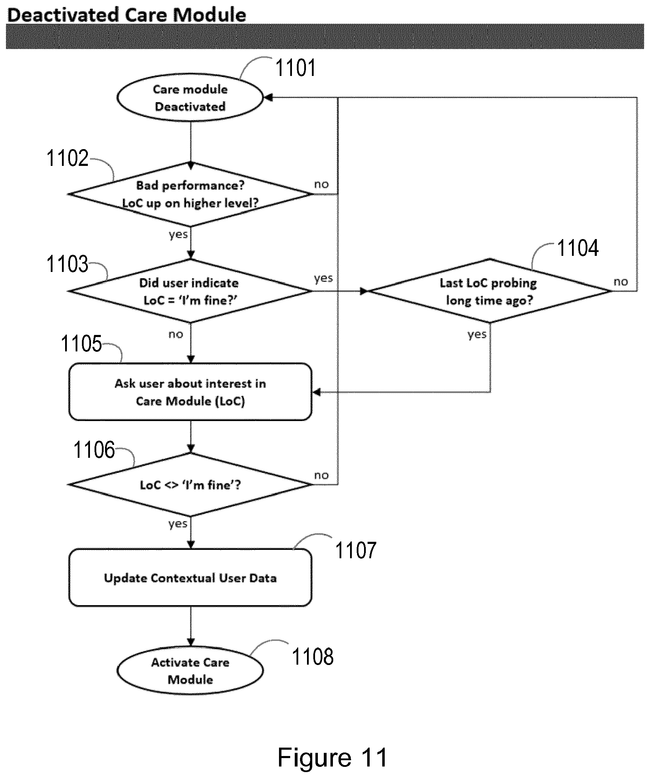

**Fig. 11** is a flowchart illustrated in a method associated with a deactivated Care Module, according to an embodiment. In some embodiments, the method as shown in Fig. 11 may be performed by the processor 102 of the apparatus 100 as described with reference to Fig. 1. In some embodiments, the method may be performed by the Caring Home module 310 as illustrated in Fig. 3.

As will be explained in more detail below, while deactivated the Care Module keeps tracking the performance of the user (results and/or behavior) in relation to the healthcare topic associated with the Care Module. It may carefully start probing the user's interest level (represented by a concern level value) with respect to the healthcare topic, unless the user has indicated not long ago that they are fine with the healthcare topic.

In the present embodiment, the method begins at step 1101, at which a Care Module is deactivated, which may be triggered due to a low associated concern level value. The method proceeds to step 1102 at which it is determined whether there is a bad performance of an activity associated with the deactivated Care Module or whether the concern level value (level of care) is high (e.g. beyond a certain severity threshold). This determination may be based on context information acquired by the processor 102 that is related to an activity of the user, the activity being associated with the healthcare topic corresponding to the deactivated Care Module. If it is determined at step 1102 that there is no bad performance and the concern level value is not high, the method proceeds back to step 1101 where the Care Module remains deactivated; if it is determined at step 1102 that there is a bad performance or the concern level value is high, the method proceeds to step 1103 where it is determined whether the user has indicated "I'm fine" as the concern level value associated with the healthcare topic corresponding to the deactivated Care Module.

If it is determined at step 1103 that the user has indicated "I'm fine" as the concern level value, the method proceeds back to step 1104 where it is determined whether the last user enquiry ("probing") regarding level of care (concern level value) is a long time ago. This may be determined by comparing a period of time between the last user enquiry and a current time with a predetermined threshold. At step 1104, if it is determined that the last user enquiry regarding level of care is not a long time ago, the method returns to step 1101 where the Care Module remains deactivated. However, if it is determined that the last user enquiry regarding level of care is a long time ago, the method proceeds to step 1105.

Moreover, if it is determined at step 1103 that the user has not indicated "I'm fine as the concern level value, the method also proceeds to step 1105, where a concern level value for the healthcare topic associated with the deactivated Care Module is obtained by enquiring the user (e.g. via a user interface).

Subsequent to obtaining a concern level value at step 1005, it is determined at step 1106 whether the concern level value is not "I'm fine". If the determination is negative, i.e. the concern level value is "I'm fine", the method proceeds to step 1101 where the Care Module remains deactivated. If the determination is positive, i.e. the concern level value is not "I'm fine", the method proceeds to step 1107 where contextual user data is updated with respect to the newly obtained concern level value, and then to step 1108 where the Care Module is activated.

It will be appreciated that in alternative embodiments the method may not comprise all of the steps described, and one or more steps as illustrated in Fig. 11 may be omitted. It will be also be appreciated that in alternative embodiments, the steps as illustrated in Fig. 11 may be performed in a different order.

**Fig. 12** and **Fig. 13** are schematic diagrams of a dialogue scheduling unit 1200 of the apparatus of Fig. 4. Specifically, Fig. 13 shows an exemplary implementation of the dialogue scheduling unit 1200 of Fig. 12.

As shown in Fig. 12 and Fig. 13, the dialogue scheduling unit 1200 (which may be referred to as a "dialogue scheduler" as mentioned) comprises a dialogue handling unit 1210 (which may be referred to as a "dialogue handler" as shown in the drawing) and a message queue 1220. The dialogue scheduling unit 1200 may be configured to receive contest information from the context modelling unit, the user profile managing unit, and dialogue content from one or more Care Modules.

In the present embodiment, the message queue 1220 comprises a plurality of healthcare-related messages 1221, 1222, 1223, 1224, 1225, and 1226. Each of the plurality of healthcare-related messages may include dialogue content (e.g. associated with a particular Care Module) that is to be presented to a user. Specifically, each of the plurality of healthcare-related messages may contain information or a question related to one of the plurality of phases (e.g. user interest, information, proposal, execution, progress evaluation, and end result evaluation) as described with reference to the operation of the Care Engine 610 of Fig. 6. For example, a healthcare-related message may be a question regarding a degree of teeth whiteness desired by the user. The user may be able to provide a response via a user interface (e.g. by selecting one of two pictures demonstrating different degrees of teeth whiteness).

The dialogue handling unit 1210 is configured to manage the selection and timing of the healthcare-related messages in the message queue 1220, and keeps track of the following aspects:

- a current activity of the user (retrieved from the context modelling unit)
- a current topic of the conversation, which will be unset after a period of no conversation
- a burden indicator value which is indicative of a recent amount of interaction with the user, and therefore a likelihood of overloading the user with excessive information.

The burden indicator value in the present embodiment may operate in a similar manner to that as described with reference to Fig. 2. For example, the burden indicator value may be updated based on acquired context information. Therefore, for the sake of conciseness, description relating to the burden indicator value is omitted.

In some embodiments, the dialogue handling unit 1210 may be configured to determine a priority value (which may be referred to as a "relevance score") for each of the plurality of healthcare-related messages based on at least one of the acquired context information and the selected healthcare topic. The dialogue handling unit 1210 may also be configured to provide at least one of the plurality of healthcare-related messages in the message queue 1220 to the user based on the priority value. The at least one of the plurality of healthcare-related messages may be provided via a user interface (e.g. a conversational user interface). In some embodiments, providing at least one of the plurality of healthcare-related messages may be further based on a time of a respective healthcare-related message in the message queue 1220.

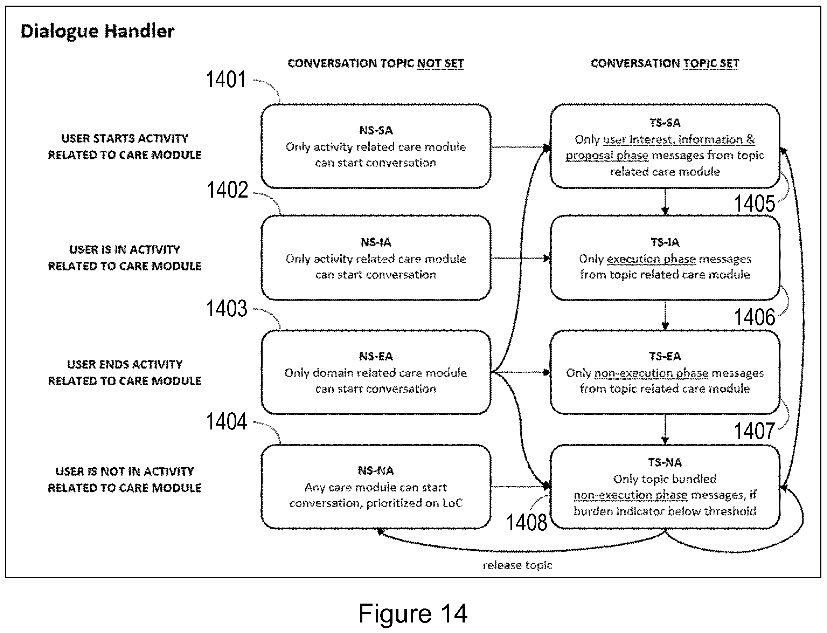

**Fig. 14** is a finite state diagram representing the behavior of the dialogue handling unit of the dialogue scheduling unit of Fig. 12 and Fig. 13.

In the present embodiment, conversations can be started by a system for providing context-based intervention to a user, e.g. the system 300 as illustrated with reference to Fig. 3, via a user interface (e.g. the conversational user interface 310 of the system 300 in Fig. 3) at any time. The type of healthcare-related messages that can be presented to a user may be restricted, dependent on an activity being performed by the user. As will be explained in more detail below, the type of healthcare-related messages that can be presented to the user may be dependent on a selected healthcare topic (i.e. an activated "Care Module" in some embodiments). In some embodiments, at least one healthcare-related messages may be at least part of an intervention recommendation provided to the user.

The finite state diagram of Fig. 14 includes a plurality of exemplary states that can be set by the system, wherein each of the plurality of states is associated with one of: "conversation topic not set" and "conversation topic set", and with one of: "starting activity", "in activity", "ending activity", and "no activity". In this case, "starting activity" corresponds to when the user starts an activity that is related to a Care Module, "in activity" corresponds to when the user is in an activity that is related to a Care Module, "ending activity" corresponds to when the user is ending an activity related to a Care Module, and "no activity" corresponds to when the user is not in an activity that is related to a Care Module.

Therefore, the plurality of states include "Topic not set, starting activity" (NS-SA) 1401, "Topic not set, in activity" (NS-IA), "Topic not set, ending activity" (NS-EA) 1403, "Topic not set, no activity" (NS-NA) 1404, "Topic set, starting activity" (TS-SA) 1405, "Topic set, in activity" (TS-IA) 1406, "Topic set, ending activity" (TS-EA) 1407, and "Topic set, no activity" (TS-NA) 1408. Further explanation relating to some of the plurality of states is provided in the following:

"Topic not set, starting activity" (NS-SA) 1401: If the user starts an activity (e.g. brushing teeth), then only healthcare-related messages that are associated with this activity (e.g. healthcare-related messages associated with the Care Module "tooth brushing" and/or healthcare-related messages associated with the Care Module "oral health") can be presented. Also, only healthcare-related messages that are associated with user interest, information and proposal are presented. If such a healthcare-related message is in a message queue, then the state of the system can be set as "topic set, starting activity" (TS-SA) 1401 and further defined by assigning "brushing teeth" or "oral health" as the healthcare topic, so as to start the relevant conversation and present the healthcare message to the user.

"Topic (not) set, in activity" (NS-IA / TS-IA) 1402, 1406: During an activity, it would be preferable not to distract the user with healthcare-related messages other than the ones that support their current activity. Therefore, during the activity the system should only allow starting or continuing activity-related conversations with healthcare-related messages that are associated with the execution of the activity. In other words, when the system is in a "topic (not) set, in activity" state, only healthcare-related messages associated with the coaching or guidance of the activity can be presented to the user (e.g. messages relating to coaching or guiding the tooth brushing activity).

"Topic not set, ending activity" (NS-EA) 1403: The end of an activity (e.g. tooth brushing) marks an important moment for:

(1) Starting a conversation on the activity that has just ended, for example discussing a state of the user, user interest for receiving coaching, evaluating progress of the coaching, or asking the user for subjective input on the activity session. In this case, subsequent to setting the state to "topic not set, ending activity" (NS-EA) 1403, the state can then be set to "topic set, ending activity" (TS-EA) 1407.

(2) Starting a conversation on an activity that is related to the activity that has just ended (e.g. by presenting a message related to the healthcare topic "tongue cleaning") or within the same domain as the activity that has just ended (e.g. by presenting a message related to the healthcare topic "oral health"). This may result in: (a) the user becoming engaged in a conversation on the new healthcare topic, and thus triggering the state of the system to be set as "topic set, no activity" (TS-NA) 1408; or (b) the user starting an activity related to the new healthcare topic during the conversation, and thus triggering the state of the system to be set as "topic set, starting activity" (TS-SA) 1405.

"Topic not set, no activity" (NS-NA) 1404: When the user is not involved in a Care Module related activity (i.e. an activity associated with a selected healthcare topic or an activity directly/indirectly causing a healthcare topic to be selected), and is not currently involved in a conversation, then a conversation related to any Care Module can be started, causing the state of the system to be set as "topic set, no activity" (TS-NA) 1408. In this state, only healthcare-related messages that are not associated with the execution of an activity can be presented to the user. In some embodiments, the "topic not set, no activity" (NS-NA) 1404 can be triggered when a conversation topic is released (indicated by the arrow "release topic") during the "topic set, no activity" (TS-NA) 1408 state.

In some embodiments, in the "topic (not) set, no activity" (NS-NA, TS-NA) a dialogue handling unit of the system e.g. the dialogue handler 1210 as illustrated with respect to Fig. 12, may be configured to organize and/or prioritize healthcare-related messages in a message queue such that:

- Healthcare-related messages that are associated with a higher level in the hierarchical structure of healthcare topics (Care Modules) are prioritized; and/or
- All healthcare-related messages that are associated with a respective healthcare topic (Care Module) are presented in a bundled manner, e.g. the messages being presented in together either simultaneously or in a sequence; and/or
- The burden indicator value remains below a predetermined threshold; and/or the healthcare topic is unselected after a predetermined period in which there is no conversation between the user and the system; and/or
- When a conversation is ongoing (i.e. "topic set"), then the dialogue handling unit is configured to prevent undesired topic switching while the user is involved in a conversation on the activity. Thus, depending on a starting point of the conversation, the state of the system may be triggered from "topic set, starting activity" (TS-SA) 1405, to "topic set, in activity" (TS-IA) 1406, and then to "topic set, ending activity" (TS-EA) 1407. Only when the activity is finished, and the conversation on the healthcare topic associated with the activity has ended, then a topic switch is allowed (in "topic set, no activity" (TS-NA) 1408).

By controlling an operation of the dialogue handling unit according to the finite state diagram as explained above, the following aspects can be ensured:

- conversations are natural and topics are not switched in an unnatural way;
- the user is approached at natural and convenient moments, given the current context the user is in (based on information retrieved from the context modelling unit);
- topics that the user is currently interested in are prioritized over topics of less interest (based on information retrieved from the user profile managing unit).

There is thus provided an improved method and apparatus for providing context-based healthcare intervention to a user, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines.

Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method for providing context-based healthcare intervention to a user, the method comprising:

   acquiring (202) context information from a plurality of sources, wherein the context information is related to at least one of: an activity of the user, a physiological status, of the user, and an environment of the user;
   updating (204), based on the acquired context information, a user profile associated with the user, wherein the user profile comprises a concern level value for each of a plurality of healthcare topics, and the concern level value is indicative of a degree of interest and/or requirement of the user to receive support on the respective healthcare topic;
   selecting (206) one of the plurality of healthcare topics based on the concern level values in the user profile; and
   providing (208), based on the selected healthcare topic, an intervention recommendation to the user, wherein the intervention recommendation is associated with proposed information related to the selected healthcare topic or a proposed adjustment for a user device.

2. The computer-implemented method according to claim 1, wherein selecting (206) a healthcare topic comprises:

   selecting a healthcare topic with an associated concern level value higher than a first predetermined threshold; or
   determining, subsequent to updating the user profile, a change in concern level value for at least one of the plurality of healthcare topics, and selecting the healthcare topic if the respective determined change in concern level value is higher than a second predetermined threshold; or
   selecting a healthcare topic that is relevant or similar to another healthcare topic with an associated concern level value higher than a predetermined threshold.

3. The computer-implemented method according to any one of the preceding claims, further comprising acquiring a persuasion style preference of the user for the selected healthcare topic, wherein providing an intervention recommendation is further based on the acquired persuasion style preference of the user for the selected healthcare topic.

4. The computer-implemented method any one of the preceding claims, wherein the proposed adjustment for a user device comprises at least one of: changing a setting of the user device, switching on the user device, and switching off the user device.

5. The computer-implemented method according to any one of the preceding claims, wherein the user profile further comprises a performance level value for each of the plurality of healthcare topics, and wherein providing an intervention recommendation is further based on the performance level value for the selected healthcare topic in the user profile.

6. The computer-implemented method according to any one of the preceding claims, further comprising:

   receiving an input from the user indicating an acceptance or a rejection of the provided intervention recommendation;
   wherein if the received input indicates an acceptance, the method further comprises executing the intervention recommendation, and
   wherein if the received input indicates a rejection, the method further comprises providing, based on the selected healthcare topic, a different intervention recommendation to the user.

7. The computer-implemented method according to claim 6, wherein if the received input indicates an acceptance, the method further comprises determining a change in effectiveness of the executed intervention over a predetermined time period.

8. The computer-implemented method according to claim 7, further comprising:

   determining a difference between the determined change in effectiveness of the executed intervention and an expected effectiveness index , wherein the expected effectiveness index represents an expected change in effectiveness of the executed intervention; and
   adjusting a parameter and/or a content of the executed intervention if the determined difference is larger than a predetermined threshold.

9. The computer-implemented method according to any one of claims 6 to 8, wherein if the received input indicates an acceptance, the method further comprises determining an overall satisfaction score indicative of a degree of satisfaction of the user with the executed intervention, wherein determining an overall satisfaction score comprises:

   determining a progress satisfaction score indicative of a degree of satisfaction of the user with respect to a progress resulting from the executed intervention;
   determining a progress orientation score indicative of a degree of willingness of the user to accept a more radical versus gentle means of persuasion;
   determining a persuasion satisfaction score indicative of a degree of satisfaction of the user with a persuasion style associated with the executed intervention; and
   determining the overall satisfaction score based on the progress satisfaction score, the progress orientation score, and the persuasion satisfaction score.

10. The computer-implemented method according to claim 9, further comprising:

    determining a difference between the overall satisfaction score and an expected satisfaction score, wherein the expected satisfaction score is indicative of a degree of expected satisfaction of the user with the executed intervention; and
    adjusting a parameter and/or a content of the executed intervention if the determined difference is larger than a predetermined threshold.

11. The computer-implemented method according to any one of the preceding claims, further comprising:

    acquiring a plurality of healthcare-related messages;
    determining a priority value for each of the plurality of healthcare-related messages based on at least one of the acquired context information and the selected healthcare topic; and
    providing at least one of the plurality of healthcare-related messages to the user based on the priority value.

**12.** The computer-implemented method according to claim 11, wherein providing at least one of the plurality of healthcare-related messages is further based on a burden indicator value, wherein the burden indicator value is indicative of a likelihood of overloading the user with excessive information.

**13.** The computer-implemented method according to claim 11 or claim 12, further comprising, subsequent to providing a healthcare-related message to the user, updating the burden indicator value.

**14.** A computer program product comprising a computer readable medium, the computer readable medium having computer readable code embodied therein, the computer readable code being configured such that, on execution by a suitable computer or processor, the computer or processor is caused to perform the method as claimed in any of the preceding claims.

**15.** An apparatus (100) for providing context-based healthcare intervention to a user, the apparatus comprising a processor (102) configured to:

acquire context information from a plurality of sources , wherein the context information is related to at least one of: an activity of the user, a physiological status, of the user, and an environment of the user;
update, based on the acquired context information, a user profile associated with the user, wherein the user profile comprises a concern level value for each of a plurality of healthcare topics, and the concern level value is indicative of a degree of interest and/or requirement of the user to receive support on the respective healthcare topic;
select one of the plurality of healthcare topics based on the concern level values in the user profile; and
provide, based on the selected healthcare topic, an intervention recommendation to the user, wherein the intervention recommendation is associated with proposed information related to the selected healthcare topic or a proposed adjustment for a user device.

Figure 1

```
┌─────────────────────────────┐
│                             │
│            202              │
│                             │
└─────────────────────────────┘
              │
              ▼
┌─────────────────────────────┐
│                             │
│            204              │
│                             │
└─────────────────────────────┘
              │
              ▼
┌─────────────────────────────┐
│                             │
│            206              │
│                             │
└─────────────────────────────┘
              │
              ▼
┌─────────────────────────────┐
│                             │
│            208              │
│                             │
└─────────────────────────────┘
```

Figure 2

Figure 3

Figure 4

EP 3 582 226 A1

EP 3 582 226 A1

**Library of Care Modules**

Figure 5

Figure 6

User profile manager

700

710

User profile controller

720

Contextual user data

Figure 7

EP 3 582 226 A1

**Contextual user data**

Personal
Care

Level of care = 2?
Performance = 3.4
Activation = active
Persuasion style pref = 2?

810

800

822

826

Oral Health
Care

Level of care = 2?
Performance = 6.4
Activation = active
Persuasion style pref = 2?

Sleep

Level of care = ?
Performance = 4.6
Activation = probing
Persuasion style pref = ?

824

...

822A

Tooth
brushing

Level of care = 3
Performance = 6.9
Activation = strong
Persuasion style pref = 2

Teeth
whitening

Level of care = 1
Performance = 7.5
Activation = inactive
Persuasion style pref = 2?

...

822C

822B

Figure 8

EP 3 582 226 A1

**Interest level** 910

| | |
|---|---|
| 3 | I need help |
| 2 | It's on my mind |
| 1 | I'm fine |

**Performance level** 920

| | |
|---|---|
| 10 | |
| 9 | It's going very well |
| 8 | |
| 7 | |
| 6 | It's going ok |
| 5 | |
| 4 | |
| 3 | |
| 2 | It's not going ok |
| 1 | |

**Activation level** 930

| | |
|---|---|
| 1 | Active |
| 0 | Inactive, checking performance |

**Persuasion style preference** 940

| | |
|---|---|
| 3 | Strong intervention |
| 2 | Active intervention |
| 1 | Careful intervention |

Figure 9

Figure 10

1001 — Care module activated

1002 — Interest (level of care) = help/worried?

1004 — Care module deactivated (no)

1005 — Determine / ask interest level (level-of-care) (unknown)

1003 — Provide level-of-care tailored information (yes)

1006 — Persuasion style preference available?

1007 — Pick persuasion style (random / based on big data + user profile) (no)

1008 — Propose persuasion style tailored intervention (yes)

1009 — User accepts proposed intervention?

1011 — Picks next best persuasion style (no)

1010 — Execute & track intervention progress (yes)

1012 — Is progress satisfactory?

1013 — Update persuasion style preference (yes)

1014 — Desired end result achieved?

## Deactivated Care Module

Figure 11

Figure 12

EP 3 582 226 A1

EP 3 582 226 A1

**Dialogue scheduler** 1200

Dialogue handler 1210

1220

Message queue

| |
CM#1 | message | relevance score=5 | time in queue=46   1221

CM#2 | message | relevance score=3 | time in queue=12   1222

CM#4 | message | relevance score=2 | time in queue=96   1223

CM#2 | message | relevance score=3 | time in queue=56   1224

CM#1 | message | relevance score=5 | time in queue=13   1225

CM#4 | message | relevance score=2 | time in queue=69   1226

Figure 13

# Dialogue Handler

Figure 14

## EUROPEAN SEARCH REPORT

**Application Number**

EP 18 17 8028

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/33314 A2 (SALUS MEDIA INC [US]; DOUGLAS PETER [US]; KRITZER ALAN [US]; LANE THOM) 10 May 2001 (2001-05-10) | 1,2,4-15 | INV.<br>G16H20/70<br>G16H20/30 |
| Y | * p.2 l.18-36, p.3 l.33-35, p.5 l.31-p.8 l.36, p.9 l.21-38, p.10 l.28-p.11 l.12, p.11 l.22-p.12 l.12, p.16 l.24-31; figure 2 * | 3 | G16H20/60<br>G16H40/67<br>G16H50/30<br>G16H50/70 |
| X | US 2016/171180 A1 (YAGNYAMURTHY SAI SARATH CHANDRA [US] ET AL) 16 June 2016 (2016-06-16) | 1,2,4-8, 14,15 | |
| Y | * [0013], [0017]-[0018], [0085]-[0090], [0097]-[0100]; figure 6 * | 3 | |
| X | WO 01/27842 A1 (WELLPARTNER INC [US]) 19 April 2001 (2001-04-19) | 1,2,4-8, 11-15 | |
| Y | * p.2 l.1-30; p.19 l.12-p.21 l.7; figure 7 * | 3 | |
| X | US 2003/225731 A1 (VIDGEN EDWARD [CA]) 4 December 2003 (2003-12-04) | 1,2, 4-10,14, 15 | TECHNICAL FIELDS SEARCHED (IPC)<br><br>G16H |
| Y | * [0006]-[0010], [0026]-[0038], [0049]; claims 1, 4-5; figures 1-2 * | 3 | |
| X | US 2018/114602 A1 (DAMANI SAMIR B [US] ET AL) 26 April 2018 (2018-04-26) | 1,2, 4-11,14, 15 | |
| Y | * [0007], [0050], [0052], [0056], [0062], [0094]-[0095], [0150]-[0151], [0170], [0173], [0192]; figures 6, 29-30 * | 3 | |

-/--

The present search report has been drawn up for all claims

| Place of search | Date of completion of the search | Examiner |
|---|---|---|
| Munich | 30 November 2018 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 17 8028

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2015/033151 A2 (ZERO360 INC [US]; KARSTEN PETER [GB]; ARRIOLA GEORGE [US]; KODERA KOUJ) 12 March 2015 (2015-03-12)<br>* p.1 l.27-p.4 l.4; p.17 l.29-33; p.18 l.5-12; p.20 l.11-p.22 l.2; p.23 l.22-33; p.25 l.13-31; p.30 l.5-22; figure 11 * | 1,2,4,5,14,15<br><br>3 | |
| X<br>Y | US 2006/074279 A1 (BROVER EVGENY [IL]) 6 April 2006 (2006-04-06)<br>* [0003]-[0004]; [0008]-[0015]; [0022]-[0023]; [0026]-[0030]; [0113]-[0116]; [0131]; [0139]; claims 43, 45 * | 1,2,4,5,14,15<br><br>3 | |
| X<br>Y | US 2013/268292 A1 (KIM HYUN-YOUNG [KR] ET AL) 10 October 2013 (2013-10-10)<br>* [0010]-[0015], [0029]-[0030], [0091], [0112], [0324] * | 1,2,4-8,14,15<br><br>3 | |
| Y | US 2018/020968 A1 (DJAJADININGRAT JOHAN PARTOMO [NL] ET AL) 25 January 2018 (2018-01-25)<br>* [0007]-[0008], [0071] [0075]-[0077] * | 3 | TECHNICAL FIELDS SEARCHED (IPC) |
| A | WO 2016/065463 A1 (2352409 ONTARIO INC [CA]; GUARDLYFF S A) 6 May 2016 (2016-05-06)<br>* p.1 par.2-3; p.2 par.4-p.3 par.1; p.10 par.1-p.12 par.1; figure 5 * | 1-15 | |
| A | US 2016/125747 A1 (CHOU KATHERINE [US] ET AL) 5 May 2016 (2016-05-05)<br>* [0011]-[0015], [0039], [0044]-[0046], [0056]-[0059] * | 1-15 | |

The present search report has been drawn up for all claims

| Place of search | Date of completion of the search | Examiner |
|---|---|---|
| Munich | 30 November 2018 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 17 8028

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2018

| Patent document cited in search report | | Publication date | Patent family member(s) | | Publication date |
|---|---|---|---|---|---|
| WO 0133314 | A2 | 10-05-2001 | AU | 3790101 A | 14-05-2001 |
| | | | WO | 0133314 A2 | 10-05-2001 |
| US 2016171180 | A1 | 16-06-2016 | NONE | | |
| WO 0127842 | A1 | 19-04-2001 | AU | 8013300 A | 23-04-2001 |
| | | | AU | 8013500 A | 23-04-2001 |
| | | | WO | 0127841 A1 | 19-04-2001 |
| | | | WO | 0127842 A1 | 19-04-2001 |
| US 2003225731 | A1 | 04-12-2003 | AU | 2003229214 A1 | 19-12-2003 |
| | | | US | 2003225731 A1 | 04-12-2003 |
| | | | WO | 03102848 A2 | 11-12-2003 |
| US 2018114602 | A1 | 26-04-2018 | US | 2014088995 A1 | 27-03-2014 |
| | | | US | 2014088996 A1 | 27-03-2014 |
| | | | US | 2014089836 A1 | 27-03-2014 |
| | | | US | 2016217266 A1 | 28-07-2016 |
| | | | US | 2018114602 A1 | 26-04-2018 |
| | | | WO | 2014047570 A1 | 27-03-2014 |
| WO 2015033151 | A2 | 12-03-2015 | EP | 3042324 A2 | 13-07-2016 |
| | | | EP | 3042327 A2 | 13-07-2016 |
| | | | EP | 3042328 A2 | 13-07-2016 |
| | | | EP | 3042329 A2 | 13-07-2016 |
| | | | EP | 3042363 A1 | 13-07-2016 |
| | | | US | 2017000348 A1 | 05-01-2017 |
| | | | US | 2017003845 A1 | 05-01-2017 |
| | | | US | 2017004459 A1 | 05-01-2017 |
| | | | US | 2017004685 A1 | 05-01-2017 |
| | | | US | 2017031449 A1 | 02-02-2017 |
| | | | WO | 2015033151 A2 | 12-03-2015 |
| | | | WO | 2015033152 A2 | 12-03-2015 |
| | | | WO | 2015033153 A2 | 12-03-2015 |
| | | | WO | 2015035098 A2 | 12-03-2015 |
| | | | WO | 2015063449 A1 | 07-05-2015 |
| US 2006074279 | A1 | 06-04-2006 | NONE | | |
| US 2013268292 | A1 | 10-10-2013 | AU | 2013205245 A1 | 24-10-2013 |
| | | | BR | 102013008131 A2 | 16-06-2015 |
| | | | CN | 103366323 A | 23-10-2013 |
| | | | EP | 2648123 A1 | 09-10-2013 |
| | | | JP | 2013218699 A | 24-10-2013 |
| | | | KR | 20130113893 A | 16-10-2013 |
| | | | RU | 2013115307 A | 10-10-2014 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 17 8028

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2018

| Patent document cited in search report | Publication date | Patent family member(s) | Publication date |
|---|---|---|---|
| | | US 2013268292 A1 | 10-10-2013 |
| | | US 2018301209 A1 | 18-10-2018 |
| | | WO 2013154294 A1 | 17-10-2013 |
| US 2018020968 A1 | 25-01-2018 | CN 107106029 A | 29-08-2017 |
| | | EP 3234829 A1 | 25-10-2017 |
| | | JP 2018504684 A | 15-02-2018 |
| | | US 2018020968 A1 | 25-01-2018 |
| | | WO 2016096549 A1 | 23-06-2016 |
| WO 2016065463 A1 | 06-05-2016 | NONE | |
| US 2016125747 A1 | 05-05-2016 | EP 3016061 A1 | 04-05-2016 |
| | | US 2016125747 A1 | 05-05-2016 |

EPO FORM P0459

For more details about this annex : see Official Journal of the European Patent Office, No. 12/82

page 2 of 2